# EUROPEAN PATENT APPLICATION

(11) **EP 4 241 687 A2**
(43) Date of publication of application: **13.09.2023**
(21) Application number: 23188963.5
(22) Date of filing: 17.09.2019
(51) Int. Cl.: A61B 5/155

(54) **SYSTEMS, APPARATUS, AND METHODS FOR PREVENTING CONTAMINATION OF A BLOOD DRAW SYSTEM**

(30) Priority: 17.09.2018 US 201862732345 P
(62) Divisional of application: 19787116.3
(71) Applicant: Velano Vascular, Inc., San Francisco, CA 94133 (US)
(72) Inventor: VANDENBRINK, Evan, Burlingame, 94010 (US); LELE, Meenal, Philadelphia, 19122 (US)
(74) Representative: Gill Jennings & Every LLP

(57) **Abstract**

In some embodiments, a system includes a container assembly, a fluid access assembly, and an adapter. The adapter includes a first engagement feature and a second engagement feature. The first engagement feature of the adapter is configured to releasably engage with a cap of the container assembly and the second engagement feature of the adapter is configured to releasably engage with an engagement feature of the fluid access assembly such that, in a first configuration in which the first engagement feature of the adapter is engaged with the cap and the second engagement feature of the adapter is engaged with the engagement feature of the fluid access assembly, the cap of the container assembly is at least partially disposed within the interior of the housing and spaced from a fluid access component of the fluid access assembly.

## Description

### Cross-Reference to Related Applications

This application claims priority to and the benefit of U.S. Provisional Application No. 62/732,345, filed September 17, 2018, entitled "Systems, Apparatus, and Methods for Preventing Contamination of a Blood Draw System," the entire content of which is hereby expressly incorporated by reference for all purposes.

### Background

Embodiments described herein relate to systems, apparatus, and methods for collecting an initial volume of blood during a blood draw.

When collecting a blood specimen from an indwelling vascular access devices, like a peripheral IV catheter (PIVC), a central venous catheter (CVC), or a peripherally inserted central catheter (PICC), the first 1-10 ml of blood is often wasted (i.e., disposed of) to avoid contamination from fluids in the dead space of the vascular access path of such devices. This extra step, while reducing contamination of the blood specimen, may be forgotten or improperly performed, thus compromising the blood specimen.

Blood cultures are often used as a tool to detect the presence of bacteria or fungi in a blood sample of a patient, to identify the type of bacteria or fungi present, and to direct the treatment of the patient. Accidental contamination of the blood sample, however, is a common problem causing false positives, which often results in a patient being prescribed unnecessary treatments (e.g., broad spectrum antibiotics). To address this, some healthcare providers clean the skin of the patient prior to a blood draw procedure. While this reduces the false positive rate, the rate is still significant (e.g., 3-5%) due to bacteria and/or fungi residing in, for example, hair follicles. Therefore, some systems also divert a small volume of the initial blood drawn and discard the initial volume. These systems, however, can be expensive to operate and often rely on puncturing a patient's skin with painful needles to collect the sample.

Additionally, needle-free blood draw systems, such as PIVO^{™}, are intended to be used in conjunction with intravenous catheters disposed within the patient. These needle-free blood draw systems may be configured to receive evacuated tubes such that the evacuated tubes, when in fluidic communication with a patient's vasculature system via the needle-free blood draw system, can draw blood into a reservoir of the evacuated tube due to the pressure differential between the reservoir and the patient's vasculature. Since the evacuated tubes are often not provided in a sterile condition, every time an evacuated tube is coupled to the needle-free blood draw system, there may be a risk of contamination. Even when healthcare providers wipe the surface of a resealable membrane of an evacuated tube (e.g., with an alcohol pad), the contamination risk to the needle-free blood draw system may be too high to use the evacuated tube as a waste tube collector prior to using the needle-free blood draw system to collect a blood sample for blood culturing.

Thus, there is a need for a system and method for collecting blood samples such that an initial blood draw volume for discarding can be collected easily and efficiently without contaminating an interface of the blood draw system.

### Summary

In some embodiments, a system includes a container assembly, a fluid access assembly, and an adapter. The container assembly includes a cap and defines a reservoir. The container assembly has a first end and a second end, and the cap is disposed at the first end of the container assembly. The fluid access assembly includes a housing defining an interior, a fluid access component, a fluid connector component, and an engagement feature. The housing has a first end and a second end, the fluid access component extending from the first end of the housing into the interior of the housing. The fluid access component defines a lumen. The fluid connector component is disposed on the first end of the housing and is configured to be coupled to a patient access device such that the lumen of the fluid access component can be in fluidic communication with a patient's vasculature via the fluid connector component and the patient access device. The engagement feature of the fluid access assembly is disposed on the second end of the housing. The adapter includes a first engagement feature and a second engagement feature. The first engagement feature of the adapter is configured to releasably engage with the cap of the container assembly and the second engagement feature of the adapter is configured to releasably engage with the engagement feature of the fluid access assembly such that, in a first configuration in which the first engagement feature of the adapter is engaged with the cap and the second engagement feature of the adapter is engaged with the engagement feature of the fluid access assembly, the cap of the container assembly is at least partially disposed within the interior of the housing and spaced from the fluid access component.

### Brief Description of the Drawings

FIG. 1 is a schematic illustration of a system, according to an embodiment.
FIGS. 2A and 2B are perspective views of a system in an assembled configuration and an exploded configuration, respectively, according to an embodiment.
FIG. 2C is a top view of the system of FIG. 2A.
FIG. 2D is a perspective view of a portion of the system of FIG. 2A.
FIG. 2E is a side view of the system of FIG. 2A in an initial configuration.
FIGS. 2F-2I are perspective views of the system of FIG. 2A in various stages of operation.
FIGS. 3A and 3B are perspective views of a system in an assembled configuration and an exploded configuration, respectively, according to an embodiment.
FIG. 3C is bottom view of the system of FIG. 3A.
FIGS. 4A and 4B are perspective views of a system in an assembled configuration and an exploded configuration, respectively, according to an embodiment.
FIGS. 5A and 5B are perspective views of a system in an assembled configuration and an exploded configuration, respectively, according to an embodiment.
FIGS. 6A and 6B are perspective views of a system in a first configuration from a first angle and a second angle, respectively, according to an embodiment.
FIG. 6C is a perspective view of the system of FIG. 6A in a second configuration.
FIG. 6D is a perspective view of the system of FIG. 6A in a third configuration.
FIG. 7 is a perspective view of a system in a first configuration, according to an embodiment.
FIG. 8A is a perspective view of an adapter, according to an embodiment.
FIG. 8B is a cross-sectional view of a system including the adapter of FIG. 8A in a first configuration, according to an embodiment.
FIG. 8C is a close up view of a first portion of the system of FIG. 8B.
FIG. 8D is a close up view of a second portion of the system of FIG. 8B.
FIG. 8E is a cross-sectional view of the system of FIG. 8B in a second configuration.
FIG. 9 is a flow chart of a method, according to an embodiment.
FIG. 10A is an exploded perspective view of a system, according to an embodiment.
FIG. 10B is a distal end view of an adapter of the system of FIG. 10A.
FIGS. 10C-10E are side views of the system of FIG. 10A in various stages of assembly.
FIG. 10F is a cross-sectional side view of the system of FIG. 10A in an assembled configuration.
FIG. 10G is a perspective view of the system of FIG. 10A in an assembled configuration.
FIG. 10H is a side view of the system of FIG. 10A in a second configuration.
FIG. 10I is a side view of the system of FIG. 10A during the transition from the second configuration of FIG. 10H to a third configuration.

### Detailed Description

In some embodiments, a system includes a container assembly, a fluid access assembly, and an adapter. The container assembly includes a cap and defines a reservoir. The container assembly has a first end and a second end, and the cap is disposed at the first end of the container assembly. The fluid access assembly includes a housing defining an interior, a fluid access component, a fluid connector component, and an engagement feature. The housing has a first end and a second end, the fluid access component extending from the first end of the housing into the interior of the housing. The fluid access component defines a lumen. The fluid connector component is disposed on the first end of the housing and is configured to be coupled to a patient access device such that the lumen of the fluid access component can be in fluidic communication with a patient's vasculature via the fluid connector component and the patient access device. The engagement feature of the fluid access assembly is disposed on the second end of the housing. The adapter includes a first engagement feature and a second engagement feature. The first engagement feature of the adapter is configured to releasably engage with the cap of the container assembly and the second engagement feature of the adapter is configured to releasably engage with the engagement feature of the fluid access assembly such that, in a first configuration in which the first engagement feature of the adapter is engaged with the cap and the second engagement feature of the adapter is engaged with the engagement feature of the fluid access assembly, the cap of the container assembly is at least partially disposed within the interior of the housing and spaced from the fluid access component.

In some embodiments, a method includes providing a system in an initial configuration. The system includes a container assembly including a cap and defining a reservoir. The container assembly having a first end and a second end, the cap disposed at the first end of the container assembly. The system also includes a fluid access assembly including a housing defining an interior, a fluid access component, a fluid connector component, and an engagement feature. The housing has a first end and a second end and the fluid access component extends from the first end of the housing into the interior of the housing. The fluid access component defines a lumen. The fluid connector component is disposed on the first end of the housing and is configured to be coupled to a patient access device such that the lumen of the fluid access component is in fluidic communication with a patient's vasculature via the fluid connector component and the patient access device. The engagement feature of the fluid access assembly is disposed on the second end of the housing. The system also includes an adapter including a first engagement feature and a second engagement feature. The first engagement feature of the adapter may be releasably engaged with the cap of the container assembly and the second engagement feature of the adapter may be releasably engaged with the engagement feature of the fluid access assembly in the initial configuration such that the cap of the container assembly is at least partially disposed within the interior of the housing and spaced from the fluid access component. The container assembly may be translated toward the first end of the fluid access assembly and relative to the adapter such that the cap is disengaged from the first engagement feature of the adapter and the fluid access component pierces a resealable membrane of the cap such that the reservoir of the container assembly is in fluidic communication with the fluid connector component via the lumen of the fluid access component. The second engagement feature of the adapter may be decoupled from the engagement feature of the fluid access assembly. The container assembly can be translated away from the first end of the fluid access assembly and out of the interior of the fluid access assembly such that the container assembly and the adapter are separated from the fluid access assembly.

In some embodiments, a system includes a fluid access assembly and a container assembly. The fluid access assembly includes a housing defining an interior, a fluid access component, a fluid connector component, and an engagement feature. The housing has a first end and a second end. The fluid access component extends from the first end of the housing into the interior of the housing. The fluid access component defines a lumen. The fluid connector component is disposed on the first end of the housing and is configured to be coupled to a patient access device such that the lumen of the fluid access component can be in fluidic communication with a patient's vasculature via the fluid connector component and the patient access device. The engagement feature of the fluid access assembly is disposed on the second end of the housing. The container assembly includes a cap and an engagement feature. The container assembly defines a reservoir and has a first end and a second end. The cap is disposed at the first end of the container assembly. The engagement feature of the container assembly is configured to releasably engage with the engagement feature of the fluid access assembly such that, in a first configuration in which the engagement feature of the adapter is engaged with engagement feature of the fluid access assembly, the cap is at least partially disposed within the interior of the housing and spaced from the fluid access component.

In some embodiments, a method includes providing a system in an initial configuration. The system includes a fluid access assembly and a container assembly. The fluid access assembly includes a housing defining an interior, a fluid access component, a fluid connector component, and an engagement feature. The housing has a first end and a second end, the fluid access component extending from the first end of the housing into the interior of the housing. The fluid access component defines a lumen. The fluid connector component is disposed on the first end of the housing and configured to be coupled to a patient access device such that the lumen of the fluid access component can be in fluidic communication with a patient's vasculature via the fluid connector component and the patient access device. The engagement feature of the fluid access assembly is disposed on the second end of the housing. The container assembly includes a cap and an engagement feature. The container assembly defines a reservoir and has a first end and a second end. The cap is disposed at the first end of the container assembly. The engagement feature of the container assembly is configured to releasably engage with the engagement feature of the fluid access assembly such that, in the initial configuration in which the engagement feature of the adapter is engaged with the engagement feature of the fluid access assembly, the cap is at least partially disposed within the interior of the housing and spaced from the fluid access component. The container assembly can be translated toward the first end of the fluid access assembly such that the engagement feature of the container assembly is disengaged from the engagement feature of the fluid access assembly and the fluid access component pierces a resealable membrane of the cap such that the reservoir of the container assembly is in fluidic communication with the fluid connector component via the lumen of the fluid access component. The container assembly can be translated away from the first end of the fluid access assembly and out of the interior of the fluid access assembly such that the container assembly is separated from the fluid access assembly.

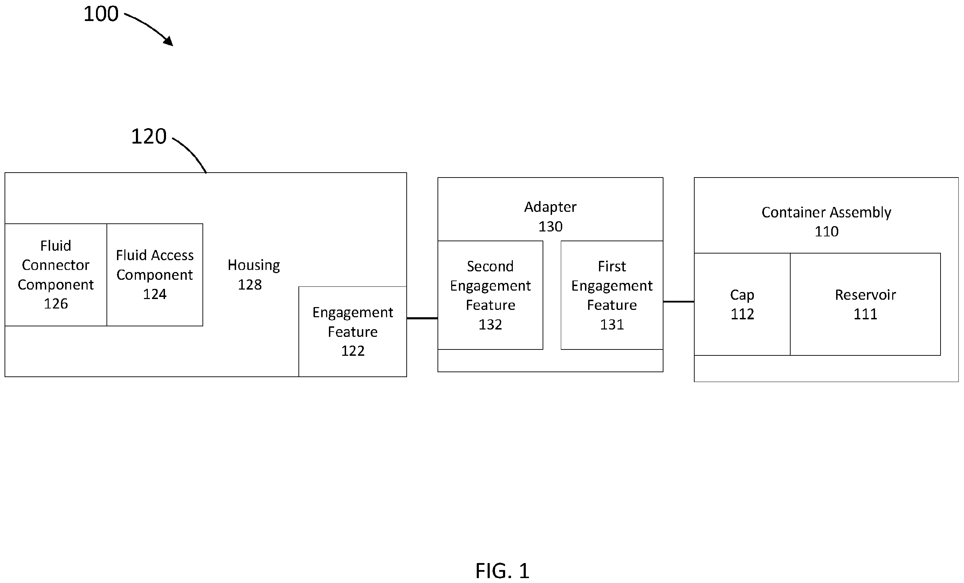

FIG. 1 is a schematic illustration of a system 100. As shown in FIG. 1, the system 100 includes a container assembly 110, a fluid access assembly 120, and an adapter 130. The container assembly 110 can include a cap 112 and can define a reservoir 111. The fluid access assembly 120 can include a housing 128 defining an interior, an engagement feature 122, a fluid access component 124, and a fluid connector component 126. The adapter 130 can include a first engagement feature 131 and a second engagement feature 132. The first engagement feature 131 of the adapter 130 can be configured to releasably engage with the cap 112 of the container assembly 110. The second engagement feature 132 of the adapter 130 can be configured to releasably engage with the engagement feature 122. The first engagement feature 131 of the adapter 130 can be or include any suitable engagement mechanism configured to temporarily, or releasably, hold the cap 112 in a position relative to the adapter 130 and to release the cap 112 upon a movement of the cap 112 relative to the first engagement feature 131. The movement can include, for example, a translational movement, a rotational movement, and/or a helical movement. The second engagement feature 132 of the adapter 130 and the engagement feature 122 of the fluid access assembly 120 can be or include any suitable engagement mechanism configured to temporarily, or releasably, engage with each other to temporarily hold the adapter 130 in a position relative to the fluid access assembly 120 or a component of the fluid access assembly 120 (e.g., the housing 128) and to release the adapter 130 from the fluid access assembly 120 (e.g., via a deformation of the adapter 130 resulting from the release of the cap 112 from the adapter 130 and/or via a deformation and/or movement (e.g., rotational, helical, and/or translational) of the second engagement feature 132 and/or the engagement feature 122 relative to the other of the second engagement feature 132 or the engagement feature 122 such that the second engagement feature 132 can be separated from the engagement feature 122).

The container assembly 110 can have a first end and a second end. The cap 112 can be disposed at the first end of the container assembly. The container assembly 110 can include a tube having an open end and a closed end opposite the open end. The cap 112 can be coupled to the open end such that the cap 112 and the tube define the reservoir 111. The cap 112 can include a resealable membrane. The resealable membrane may be configured such that a fluid access component, such as fluid access component 124, can pierce the resealable membrane to achieve fluidic communication with the reservoir 111. The resealable membrane of the cap 112 can be configured to reseal upon decoupling the fluid access component 124 from the cap 112 such that the reservoir 111 is fluidically isolated from an area external to the container assembly 110. The cap 112 can include ridges and/or one or more flanges disposed on an external surface of the cap 112. The reservoir 111 can be an evacuated reservoir such that, upon the reservoir 111 being placed in fluidic communication with a source of fluid (e.g., via piercing the resealable membrane of the cap 112 with a fluid access component fluidically coupled to a patient's vasculature), fluid (e.g., blood) can be drawn into the reservoir 111 due to a pressure differential between the reservoir 111 and the source of fluid. In some embodiments, the container assembly 110 can be an evacuated tube. In some embodiments, the container assembly 110 can be any suitable standard evacuated tube, such as, for example, a BD Vacutainer^{®} and/or a Greiner Bio-one^{®} VACUETTE^{®}. The cap 112 can be formed of, for example, rubber. The tube can be formed of, for example, plastic.

The fluid access assembly 120 can have a first end and a second end. The fluid access component 124 can be disposed within the interior of the housing 128 and can extend from the first end of the housing 128 into the interior of the housing 128. For example, in some embodiments, the fluid access component 124 can have a first end and a second end opposite the first end. The first end of the fluid access component 124 can be coupled to the first end of the housing 128 and the second end can be disposed in the interior of the housing 128. In some embodiments, the fluid access component 124 can include a needle defining a lumen. In some embodiments, the fluid access assembly 120 can include a flexible sheath configured to be translated relative to the needle such that a second end of the needle can be exposed.

The fluid connector component 126 can be disposed on and/or coupled to the first end of the housing 128. For example, the housing 128 can define an outlet fluidically coupled to the lumen of the fluid access component 124 to which the fluid connector component 126 can be coupled. In some embodiments, the fluid connector component 126 can include any suitable component configured to couple the housing 128 to patient access tubing (e.g., of a patient access device) such that the lumen of the fluid access component 124 is in fluidic communication with, for example, a patient vasculature system. For example, in some embodiments, the fluid connector component 126 can be a Luer connector. In some embodiments, the fluid connector component 126 can be an outlet of the housing 128 defining a lumen. In some embodiments, the fluid connector component 126 can be monolithically formed with the housing 128. In some embodiments, the fluid connector component 126 and the fluid access component 124 can be monolithically formed. For example, the fluid connector component 126 and the fluid access component 124 can be formed as a double-ended needle. In some embodiments, the fluid connector component 126 can include a needle such that the fluid connector component 126 can be directly coupled to a patient's vasculature. In some embodiments, the second end of the housing 128 can define a fluid path and/or intermediate reservoir between the fluid connector component 126 and the fluid access component 124 such that the fluid connector component 126 and the fluid access component 124 are fluidically coupled.

The engagement feature 122 of the fluid access assembly 120 can include a flange extending perpendicularly from a central axis of the housing 128. In some embodiments, the flange can be elongated such that the flange extends farther from the central axis of the housing 128 in a first direction than in a second direction. For example, the flange can form an elongated surface of the fluid access assembly 120 disposed in a plane containing the second end of the housing 128.

In some embodiments, the fluid access assembly 120 can be any suitable standard holder, such as, for example, a Greiner Bio-one^{®} VACUETTE^{®} Blood Culture Holder, a JELCO^{®} Saf-T Holder^{®} device sold by Smiths Medical, and/or a BD Vacutainer^{®} Holder device. In some embodiments, the fluid access assembly 120 may be configured to couple to and/or otherwise engage an indwelling peripheral intravenous catheter (PIV). For example, in some embodiments, the fluid access assembly 120 can include and/or be coupled to any fluid transfer device or portion of a fluid transfer device shown and/or described in U.S. Patent No. 10,076,272 entitled "Systems and Methods for Phlebotomy Through A Peripheral IV Catheter," filed on August 26, 2014 and/or in U.S. Patent Publication No. 2017/0216564 (referred to herein as the `564 publication) entitled "Devices and Methods for Fluid Transfer Through a Placed Intravenous Catheter," filed on February 2, 2016, the disclosures of each of which are incorporated by reference herein in their entirety. For example, in some embodiments, the housing 128 and the fluid access component 124 of the fluid access assembly 120 may be the same or similar in structure and/or function to the container shroud and the needle of the '564 publication.

The first engagement feature 131 of the adapter 130 can be any suitable feature configured to releasably engage with the cap 112 of the container assembly 110. In some embodiments, the first engagement feature 131 of the adapter 130 can be an inner surface of the adapter 130 that defines a through-hole. The inner surface of the adapter 130 can have a diameter sufficiently small relative to an outermost diameter of the cap 112 such that the inner surface and the cap 112 can be engaged via a friction fit. In some embodiments, the first engagement feature 131 can include a feature corresponding to a feature on the cap 112 such that the adapter 130 and the cap 112 can be releasably engaged.

The second engagement feature 132 of the adapter 130 can be any suitable feature configured to releasably engage with the engagement feature 122 of the fluid access assembly 120. For example, the second engagement feature 132 can include two oppositely disposed tabs. A latch can be disposed on the end of each tab. Each latch can be shaped and sized to receive a portion of a flange of the engagement feature 122 of the fluid access assembly 120. In some embodiments, the adapter 130 can be rotatable relative to the fluid access assembly 120 such that the second engagement feature 132 (e.g., the latches) can be rotated out of engagement with the engagement feature 122 of the fluid access assembly 120 (e.g., a flange). In some embodiments, the second engagement feature 132 can include a number of arms (e.g., two), each of the arms having a first end coupled to a base of the adapter 130 via a flexible joint and a latch disposed on the opposite end of the arm. In some embodiments, each of the arms may be curved and may form a portion of the outer perimeter of the adaptor 130. Such an adapter can be decoupled from the engagement feature 122 of the fluid access assembly 120 via, for example, rotation and/or deformation (e.g., bending). In some embodiments, the second engagement feature 132 can include a number of tabs including latches configured to be snapped over a flange of the engagement feature 122. For example, the second engagement features 132 can include two or three latching tabs. To separate such an adapter 130 from the housing 128, a user can decouple each latching tab by pulling the latch away from the flange of the engagement feature 122 such that the tab is released from engagement with the flange of the engagement feature 122.

In some embodiments, the fluid access assembly 120 can include a container size adapter such that container assemblies 110 of various sizes and shapes can be used with (e.g., disposed within and stabilized by) the fluid access assembly 120. In some embodiments, the adapter 130 can be configured (e.g., shaped and sized) to engage with an engagement feature of the container size adapter in similar ways as described above with respect to the engagement feature 122 of the fluid access assembly 120. In some embodiments, the adapter 130 can be configured (e.g., shaped and sized) to receive a portion of the container size adapter into the second engagement feature 132 of the adapter 130 when the second engagement feature 132 of the adapter 130 is engaged with the engagement feature 122 of the fluid access assembly 120.

The system 100 has a first configuration (e.g., an initial configuration) in which the first engagement feature 131 of the adapter 130 is engaged with the cap 112 and the second engagement feature 132 is engaged with the engagement feature 122 of the fluid access assembly 120. When the first engagement feature 131 of the adapter 130 is engaged with the cap 112 and the second engagement feature 132 is engaged with the engagement feature 122 of the fluid access assembly 120, the cap 112 can be spaced away from the fluid access component 124 such that the reservoir 111 is fluidically isolated from an external environment of the container assembly 110. In the first configuration, the cap 112 can be at least partially disposed within the adapter 130 and/or the housing 128. For example, a first end of the cap 112 can be disposed within the interior of the housing 128 (either projecting from the adapter 130 or within the adapter 130). In some embodiments, the first end of the cap 112 can be disposed and retained within the adapter 130 but proximal of the housing 128 in the first configuration. In some embodiments, the system 100 can be sterilized in the first configuration and packaged for sterile transport to a user (e.g., a healthcare provider).

The system 100 has a second configuration in which the lumen of the fluid access component 124 is in fluidic communication with the reservoir 111. To transition the system 100 from the first configuration to the second configuration, the container assembly 110 can be translated toward the first end of the fluid access assembly 120 such that the cap 112 is engaged with the fluid access component 124 (e.g., the fluid access component 124 pierces the cap 112) and a portion of the fluid access component 124 is disposed within the reservoir 111. For example, in embodiments in which the container assembly 110 is engaged with the first engagement feature 131 via a friction fit, a force may be applied to the container assembly 110 to overcome the force applied by the first engagement feature 131 on the container assembly 110 (e.g., the cap 112) and translate the container assembly 110 into engagement with the fluid access component 124. In the second configuration, when the fluid connector component 126 is fluidically coupled to a patient's vasculature system, fluid (e.g., blood) can be drawn through the fluid connector component 126, through the fluid access component 124, and into the reservoir 111 of the container assembly 110.

When sufficient blood has been drawn into the reservoir 111, the system 100 can be transitioned from the second configuration to a third configuration in which the container assembly 110 and the adapter 130 are separated from the fluid access assembly 120. For example, the container assembly 110 can be translated relative to the fluid access assembly 120 such that the cap 112 is disposed near the second end of the housing 128. In response to the cap 112 being disengaged from the fluid access component 124, the reservoir 111 may be fluidically isolated from an environment external to the container assembly 110 (e.g., due to the cap 112 having a resealable membrane). The adapter 130 can then be decoupled from the fluid access assembly 120 via decoupling the second engagement feature 132 from the engagement feature 122 via, for example, rotating, unsnapping, or deforming (e.g., bending) one or more portions of the second engagement feature 132 from the engagement feature 122 of the fluid access assembly 120. The adapter 130 and the container assembly 110 may then be optionally discarded.

After removing the adapter 130 and the container assembly 110 from the fluid access assembly 120, a second container assembly can be inserted into the interior of the housing and engaged with the fluid access component 124 such that the second container assembly can draw fluid (e.g., blood) into a reservoir of the second container assembly via the fluid connector component 126 and the fluid access component 124. In some embodiments, the second container assembly can include a medium (e.g., a soybean casein digest broth) in the reservoir of the second container configured to be used to perform a blood culture when combined with the patient's blood sample in the reservoir. Any suitable number of container assemblies can be engaged with the fluid access component 124 to draw fluid from a patient for various tests.

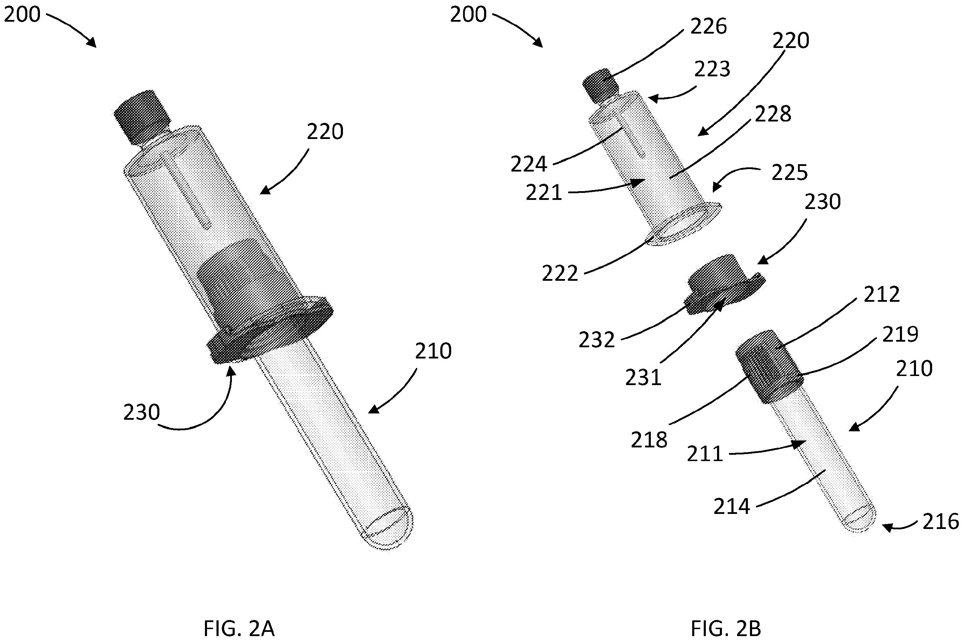

FIGS. 2A and 2B are perspective views of a system 200 in an assembled configuration and an exploded configuration, respectively. The system 200 can be the same or similar in structure and/or function to any of the systems herein, such as, for example, the system 100 described above with respect to FIG. 1. As shown in FIG. 2A, the system 200 includes a container assembly 210, a fluid access assembly 220, and an adapter 230. As shown in FIG. 2B, the container assembly 210 can include a cap 212 and a tube 214. The tube 214 can have a first end coupled to the cap 212 and a second closed end 216. The cap 212 and the tube 214 can define a reservoir 211. The fluid access assembly 220 can include a housing 228 defining an interior 221, an engagement feature 222, a fluid access component 224, and a fluid connector component 226. The adapter 230 can include a first engagement feature 231 and a second engagement feature 232. The first engagement feature 231 of the adapter 230 can be configured to releasably engage with the cap 212 of the container assembly 210. The second engagement feature 232 of the adapter 230 can be configured to releasably engage with the engagement feature 222.

The cap 212 can include a resealable membrane such that a fluid access component, such as fluid access component 224, can pierce the resealable membrane to achieve fluidic communication with the reservoir 211. The resealable membrane of the cap 212 can be configured to reseal upon decoupling the fluid access component 224 from the cap 212 such that the reservoir 211 is fluidically isolated from an area external to the container assembly 210. Furthermore, the reservoir 211 can be an evacuated reservoir such that, when the reservoir 211 is in fluidic communication with a source of fluid (e.g., via piercing the resealable membrane of the cap 212 with a fluid access component (e.g., the fluid access component 224) fluidically coupled to a patient's vasculature), fluid can be drawn into the reservoir 211 due to a pressure differential between the reservoir 211 and the source of fluid. As shown in FIG. 2B, the cap 212 can include a number of ridges 218 and an annular flange 219 disposed on an external surface of the cap 212.

The fluid access assembly 220 can have a first end 223 and a second end 225. The fluid access component 224 can be disposed within the interior 221 of the housing 228 and can extend from the first end 223 of the housing 228 into the interior 221 of the housing 228. For example, in some embodiments, the fluid access component 224 can have a first end and a second end opposite the first end, the first end of the fluid access component 224 coupled to the first end of the housing 228 and the second end of the fluid access component 224 disposed in the interior 221 of the housing 228. The fluid access component 224 can include a needle defining a lumen.

The fluid connector component 226 can be disposed on and/or be coupled to the first end 223 of the housing 228. For example, the housing 228 can define an outlet fluidically coupled to the lumen of the fluid access component 224 to which the fluid connector component 226 can be coupled. The fluid connector component 226 can include any suitable component configured to couple the housing 228 to patient access tubing such that the lumen of the fluid access component 224 is in fluidic communication with, for example, a patient vasculature system. For example, in some embodiments, the fluid connector component 226 can be a Luer connector.

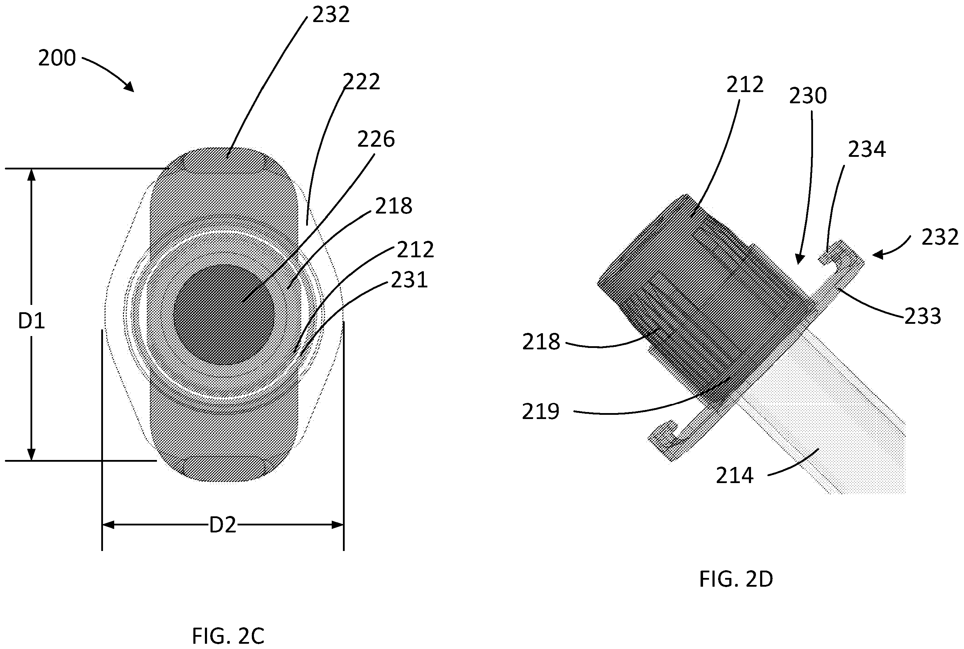

The engagement feature 222 of the fluid access assembly 220 can include a flange extending perpendicularly from a central axis of the housing 228. The engagement feature 222 can form a proximal surface of the fluid access assembly 220 and can be disposed in a plane containing the second end of the housing 228. As shown in FIG. 2C, which is a top view of the system 200, the engagement feature 222 can extend farther from the central axis of the housing 228 in a first direction than in a second direction. For example, as shown in FIG. 2C, in one direction the engagement feature 222 can have a length D1. In a second direction perpendicular to the first direction, the engagement feature 222 can have a length D2. The length D1 can be longer than the length D2.

The first engagement feature 231 of the adapter 230 can be configured to releasably engage with the cap 212 of the container assembly 210. The first engagement feature 231 of the adapter 230 can include an inner surface of the adapter 230, the inner surface defining a through-hole. A central axis of the through-hole can be configured to be coaxial with a central axis of the housing 228 of the fluid access assembly 220. As shown in FIG. 2D, which is a perspective view of the adapter 230 and a portion of the container assembly 210, the inner surface of the adapter 230 can have a diameter sufficiently small relative to an outermost diameter of the annular flange 219 of the cap 212 such that the inner surface and the cap 212 can be engaged via a friction fit. In some embodiments, the inner surface of the adapter 230 can have a diameter sufficiently small relative to an outermost diameter of the ridges 218 the cap 212 such that the inner surface and the ridges 218 of the cap 212 can be engaged via a friction fit.

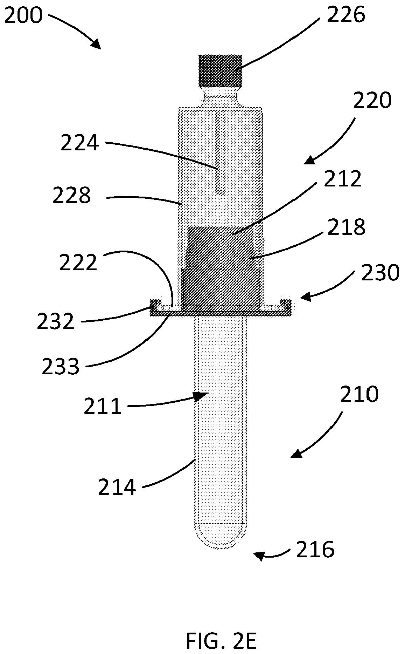

The second engagement feature 232 of the adapter 230 can be configured to releasably engage with the engagement feature 222 of the fluid access assembly 220. For example, the second engagement feature 232 can include two oppositely disposed tabs 233. A latch 234 can be disposed on the end of each tab 233. As shown, for example, in FIG. 2E, which is a side view of the system 200 in a first configuration, each latch 234 can be shaped and sized to receive a portion of the flange of the engagement feature 222 of the fluid access assembly 220.

To disengage the adapter 230 from the fluid access assembly 220, the adapter 230 can be rotatable relative to the fluid access assembly 220 such that the second engagement feature 232 (e.g., the latches) can be rotated out of engagement with the engagement feature 222 (e.g., flanges) of the fluid access assembly 220. For example, as shown in FIG. 2C, the engagement features 232 of the adapter 230 can be sized to engage with a portion of the engagement feature 222 having a length D1 in a first configuration. The adapter 230 can be rotated such that the engagement features 232 can disengage from the portion of the engagement feature 222 having a length D1 and instead be aligned with the portion of the engagement feature 222 having a length D2. Since the length D2 is shorter than the distance between the latches 232, the latches 232 will not be engaged with the engagement feature 222 after being rotated and the adapter 230 can then be separated from the container assembly 210.

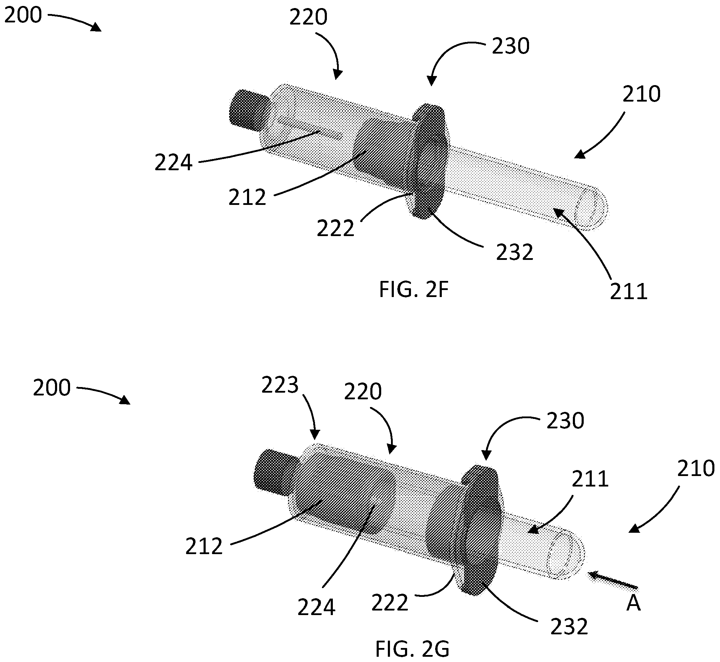

FIGS. 2F-2I are perspective views of the system 200 in various stages of use. As shown in FIG. 2F, the system 200 has a first configuration (e.g., an initial configuration) in which the first engagement feature 231 of the adapter 230 is engaged with the cap 212 and the second engagement feature 232 is engaged with the engagement feature 222 of the fluid access assembly 220. When the first engagement feature 231 of the adapter 230 is engaged with the cap 212 and the second engagement feature 232 is engaged with the engagement feature 222 of the fluid access assembly 220, the cap 212 can be spaced away from the fluid access component 224 such that the reservoir 211 is fluidically isolated from an external environment of the container assembly 210. In the first configuration, the cap 212 can be at least partially disposed within the adapter 230 and the housing 228. A first end of the cap 212 (e.g., the end opposite the surface of the cap 212 partially defining the reservoir 211) can be disposed within the interior 221 of the housing 228. In some embodiments, the system 200 can be sterilized in the first configuration and packaged for sterile transport to a user (e.g., a healthcare provider).

As shown in FIG. 2G, the system 200 can be transitioned to a second configuration in which the lumen of the fluid access component 224 is in fluidic communication with the reservoir 211. To transition the system 200 from the first configuration to the second configuration, the container assembly 210 can be translated in the direction of arrow A toward the first end 223 of the fluid access assembly 220 such that the cap 212 is engaged with the fluid access component 224 (e.g., the fluid access component 224 pierces the cap 212) and a portion of the fluid access component 224 is disposed within the reservoir 211. In the second configuration, when the fluid connector component 226 is fluidically coupled to a patient's vasculature system, fluid (e.g., blood) can be drawn through the fluid connector component 226, through the fluid access component 224, and into the reservoir 211 of the container assembly 210 (e.g., due to the pressure differential between the reservoir 211 of the container assembly 210 and the patient's vasculature system).

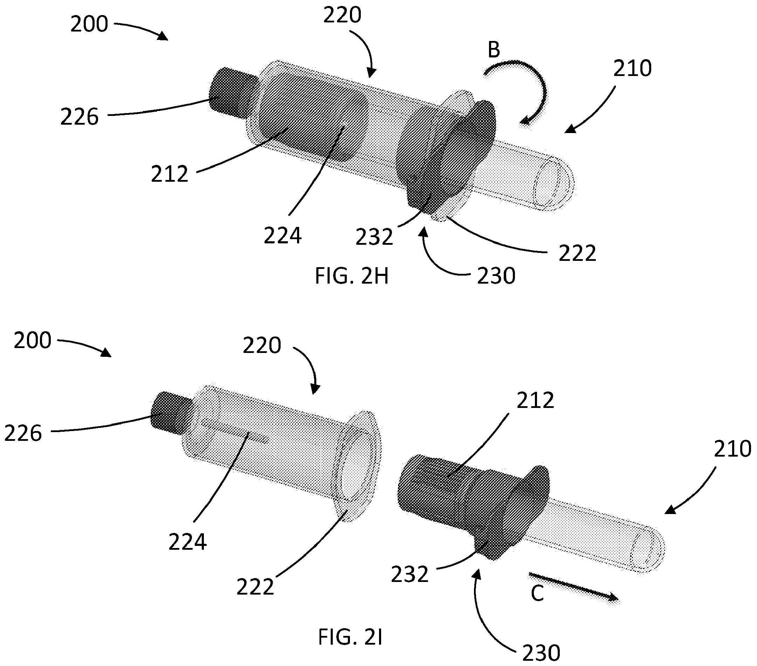

As shown in FIG. 2H, when sufficient blood has been drawn into the reservoir 211, the system 200 can be transitioned from the second configuration to a third configuration in which the second engagement feature 232 of the adapter 230 is disengaged from the engagement feature 222 of the fluid access assembly 220. In some embodiment, the amount of blood drawn into the reservoir 211 is sufficient to capture any bacteria or fungus from the fluid path that may be drawn through the fluid access assembly 220 during the initial blood draw (e.g., due to biofilm build up within the patient). For example, the amount of blood drawn may be between 0.1 and 10 ml. In some embodiments, the amount of blood drawn may be between 0.1 and 1 ml. In some embodiments, the amount of blood drawn may be between 1 and 10 ml. The adapter 230 can be rotated in the direction of arrow B relative to the fluid access assembly 220 such that the second engagement feature 232 of the adapter 230 disengages from the engagement feature 222.

As shown in FIG. 2I, after the second engagement feature 232 of the adapter 230 is disengaged from the engagement feature 222 of the fluid access assembly 220, the container assembly 210 and the adapter 230 can be separated from the fluid access assembly 220. The container assembly 210 can be translated in the direction of arrow C relative to the fluid access assembly 220 such that the cap 212 is disengaged from the fluid access component 224 such that the reservoir 211 is again fluidically isolated from an environment external to the container assembly 210 and the adapter 230 and the container assembly 210 can be decoupled from the fluid access assembly 220. The adapter 230 and the container assembly 210 can then be discarded. In some embodiments, the container assembly 210 may be translated a distance in the direction of arrow C before the adapter 230 is rotated in the direction of arrow B and disengaged from the engagement feature 222.

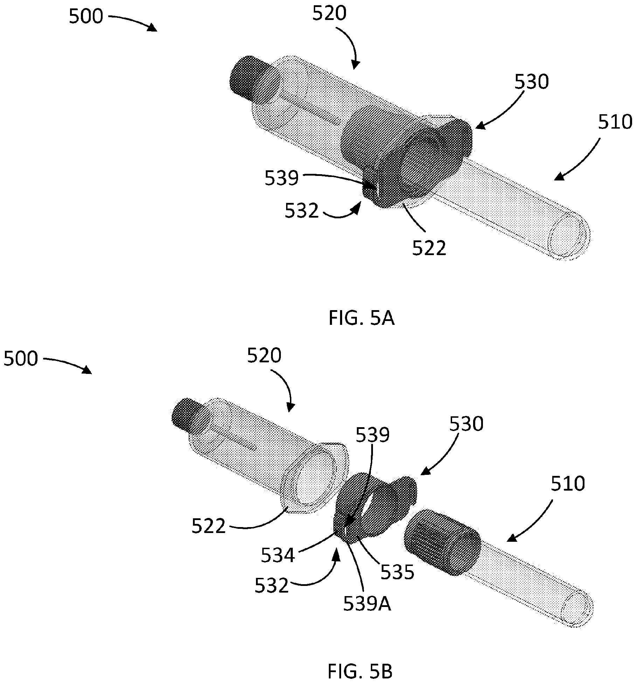

In some embodiments, the adapter can define a slot between each tab and latch. For example, FIGS. 5A and 5B are a perspective assembled view and an exploded assembly view of a system 500, respectively. The system 500 can be the same or similar in structure and/or function to any of the systems described herein, such as, for example, the system 200. For example, the container assembly 510 can be the same or similar in structure and/or function to the container assembly 210 and will not be further described herein. The fluid access assembly 520 can be the same or similar in structure and/or function to the fluid access assembly 210 and will not be further described herein. The adapter 530 can be similar in structure and/or function to the adapter 230 described above. For example, the adapter 530 can include a second engagement feature 532 including two oppositely disposed and oppositely extending tabs 535. Each tab 535 can be coupled to a latch 534 via a flexible hinge 539A. The adapter 530 can define a slot 539 between each latch 534 and tab 535. Thus, rather than the entire adapter 530 being rotated relative to the engagement feature 522 of the fluid access assembly 520, the latch 534 can be rotated relative to the tab 535 via the flexible hinge 539A such that the adapter 530 can be separated from the fluid access assembly 520. For example, each latch 534 can be rotated about an axis running through the flexible hinge 539A that is disposed parallel to a central axis of the adapter 530 and/or can be rotated relative to a plane within which the proximal surface of the tabs 535 are disposed such that the adaptor 530 may be translated away from the fluid access assembly 520 (e.g., via a user pulling on the adaptor 530 relative to the fluid access assembly 520).

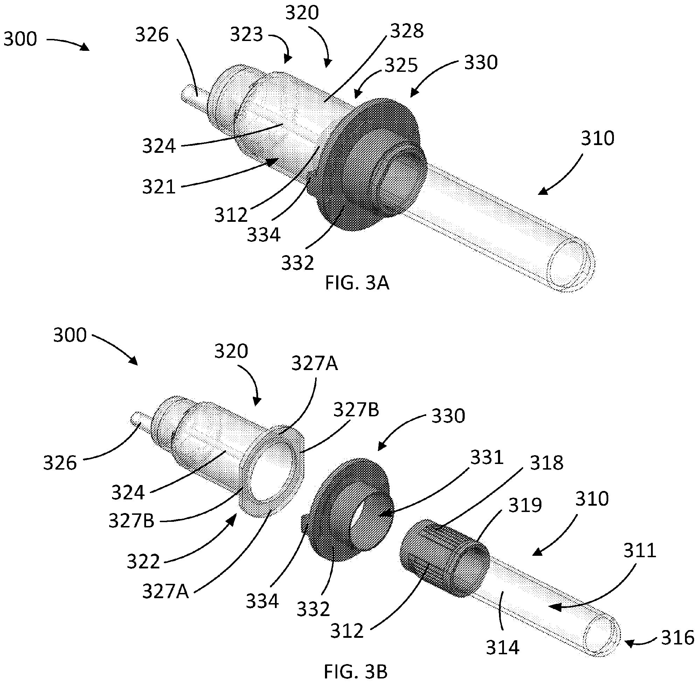

FIGS. 3A and 3B are a perspective assembled view and an exploded assembled view of a system 300, respectively. The system 300 can be the same or similar in structure and/or function to any of the systems herein, such as, for example, the system 100 described above with respect to FIG. 1 and/or the system 200. As shown in FIG. 3A, the system 300 includes a container assembly 310, a fluid access assembly 320, and an adapter 330. As shown in FIG. 3B, the container assembly 310 can include a cap 312 and a tube 314. The tube 314 can have a first end coupled to the cap 312 and a second closed end 316. The cap 312 and the tube 314 can define a reservoir 311. The fluid access assembly 320 can include a housing 328 defining an interior 321, an engagement feature 322, a fluid access component 324, and a fluid connector component 326. The adapter 330 can include a first engagement feature 331 and a second engagement feature 332. The first engagement feature 331 of the adapter 330 can be configured to releasably engage with the cap 312 of the container assembly 310. The second engagement feature 332 of the adapter 330 can be configured to releasably engage with the engagement feature 322.

The cap 312 can include a resealable membrane such that a fluid access component, such as fluid access component 324, can pierce the resealable membrane to achieve fluidic communication with the reservoir 311. The resealable membrane of the cap 312 can be configured to reseal upon decoupling the fluid access component 324 from the cap 312 such that the reservoir 311 is fluidically isolated from an area external to the container assembly 310. Furthermore, the reservoir 311 can be an evacuated reservoir such that, when the reservoir 311 is in fluidic communication with a source of fluid (e.g., via piercing the resealable membrane of the cap 312 with a fluid access component fluidically coupled to a patient's vasculature), fluid can be drawn into the reservoir 311 due to a pressure differential between the reservoir 311 and the source of fluid. As shown in FIG. 3B, the cap 312 can include ridges 318 and an outwardly projecting annular flange 319 disposed on an external surface of the cap 312. The cap 312 can be formed of, for example, rubber. The tube 314 can be formed of, for example, plastic.

The fluid access assembly 320 can have a first end 323 and a second end 325. The fluid access component 324 can be disposed within the interior 321 of the housing 328 and can extend from the first end 323 of the housing 328 into the interior 321 of the housing 328. For example, in some embodiments, the fluid access component 324 can have a first end and a second end opposite the first end, the first end of the fluid access component 324 coupled to the first end of the housing 328 and the second end of the fluid access component 324 disposed in the interior 321 of the housing 328. The fluid access component 324 can include a needle defining a lumen.

The fluid connector component 326 can be coupled to the first end 323 of the housing 328. For example, the fluid connector component 326 can be coupled to the housing 328 and can define an outlet fluidically coupled to the lumen of the fluid access component 324. The fluid connector component 326 can include any suitable component configured to couple the housing 328 to patient access tubing such that the lumen of the fluid access component 324 is in fluidic communication with, for example, a patient vasculature system. In some embodiments, the fluid connector component 326 can be monolithically formed with the housing 328.

The engagement feature 322 of the fluid access assembly 320 can include a flange extending perpendicularly from a central axis of the housing 328. The engagement feature 322 can form an elongated surface of the fluid access assembly 320 disposed in a plane containing the second end of the housing 328. As shown in FIG. 3B, the engagement feature 322 can include portions (i.e., flange extensions 327A) that extend farther from the central axis of the housing 328 than other portions (i.e., flange portions 327B) of the engagement feature 322 extend from the central axis of the housing 328. The flange extensions 327A can be oppositely disposed and can each have a curved outer edge. The flange portions 327B can be oppositely disposed and can each have a straight outer edge. The flange extensions 327A can be separated from each other by the flange portions 327B. In some embodiments, the fluid access assembly 320 can be any suitable standard holder, such as, for example, a Greiner Bio-one^{®} VACUETTE^{®} Blood Culture Holder.

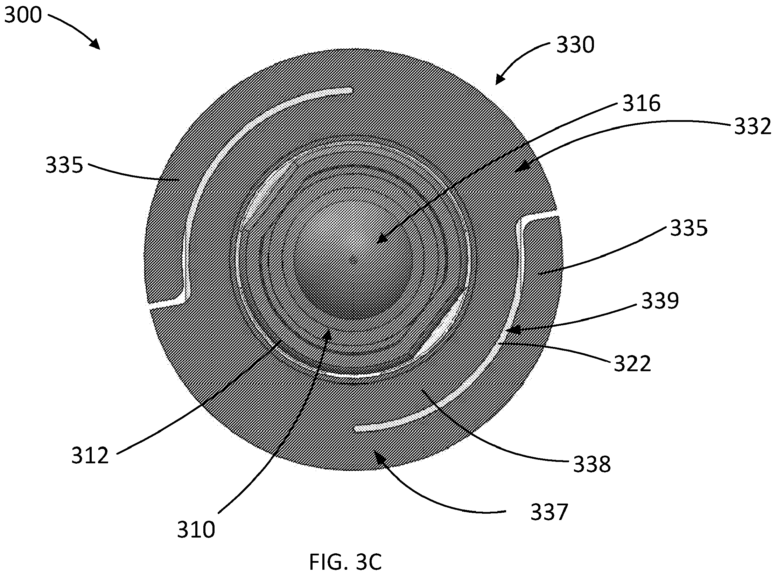

The first engagement feature 331 of the adapter 330 can be configured to releasably engage with the cap 312 of the container assembly 310. The first engagement feature 331 of the adapter 330 can include an inner surface of the adapter 330, the inner surface defining a through-hole. A central axis of the through-hole can be configured to be coaxial with a central axis of the housing 328 of the fluid access assembly 320. As shown in FIG. 3C, which is a bottom view of the system 300, the inner surface of the adapter 330 can have a diameter sufficiently small relative to an outermost diameter of the outwardly projecting annular flange 319 of the cap 312 such that the inner surface and the cap 312 can be engaged via a friction fit. In some embodiments, the inner surface of the adapter 330 can have a diameter sufficiently small relative to an outermost diameter of the ridges 318 the cap 312 such that the inner surface and the ridges 318 of the cap 312 can be engaged via a friction fit.

The second engagement feature 332 of the adapter 330 can be configured to releasably engage with the engagement feature 322 of the fluid access assembly 320. For example, the second engagement feature 332 can include two curved arms 335. Each curved arm 335 has a first end coupled to a base 338 of the adapter 330 via a flexible joint 337. The second end of each curved arm 335 includes a latch 334, as can be seen in FIGS. 3A and 3B. As can be seen in FIG. 3C, the adapter 330 can have an overall outer circular profile, and each of the curved arms 335 and the base 338 can define a slot 339. In some embodiments, the latches 334 can be configured to receive the flange extensions 327A and can be separated from the flange extensions 327A via rotating the adapter 330 relative to the engagement feature 322 until the latches 334 are disengaged from the flange extensions 327A and then the adapter 330 can be pulled proximally away from the first end 323 of the housing 328. In some embodiments, the latch 334 can be configured to receive the flange portions 327B, and the adapter 330 can be separated from the engagement feature 322 via rotating the container assembly 310 relative to the housing 328 sufficiently such that the curved arms 335 are disengaged from the flange portions 327B via being flexed out of engagement through bending the flexible joint 337. For example, each latch 334 can be rotated about an axis running through the flexible joint 337 that is disposed parallel to a central axis of the adapter 330 and/or can be rotated relative to a plane within which the proximal surface of the base 338 are disposed such that the adaptor 330 may be translated away from the fluid access assembly 320 (e.g., via a user pulling on the adaptor 330 relative to the fluid access assembly 320). The adapter 330 can then be separated from the container assembly 310.

Although the adapter 330 is shown and described as including two curved arms 335, in some embodiments the adapter 330 may include any suitable number of arms having any suitable shape. For example, the adapter 330 may include three or four arms, each arm having a first end coupled to a base similar to the base 338 via a flexible joint similar to the joint 337 and a second end including a latch similar to latch 334. In some embodiments, the arms 335 and the base 338 may be configured such that the arms 335 are straight and extend away from the base 338 in a substantially straight line.

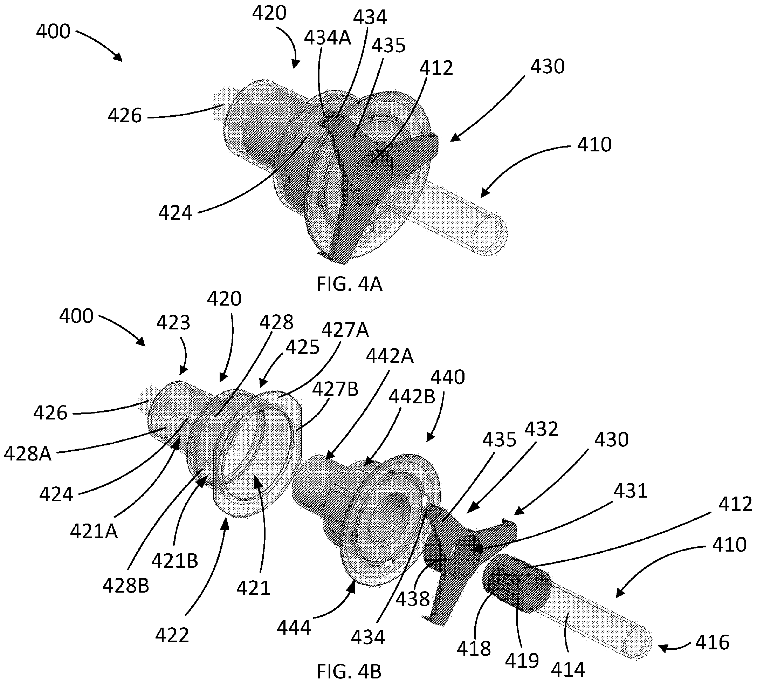

FIGS. 4A and 4B are a perspective assembled view and an exploded assembled view of a system 400, respectively. The system 400 can be the same or similar in structure and/or function to any of the systems herein, such as, for example, the system 100 described above with respect to FIG. 1 and/or the system 200. As shown in FIG. 4A, the system 400 includes a container assembly 410, a fluid access assembly 420, and an adapter 430. The system 400 can also include container size adapter 440. As shown in FIG. 4B, the container assembly 410 can include a cap 412 and a tube 414. The tube 414 can have a first end coupled to the cap 412 and a second closed end 416. The cap and the tube 414 can define a reservoir 411. The fluid access assembly 420 can include a housing 428 defining an interior 421, an engagement feature 422, a fluid access component 424, and a fluid connector component 426. The housing 428 can have a stepped outer profile such that the housing 428 includes a first inner diameter portion 428A and a second inner diameter portion 428B. The first inner diameter portion 428A can define a first portion 421A of the interior 421 and the second inner diameter portion 428B can define a second portion 421B of the interior 421. The adapter 430 can include a first engagement feature 431 and a second engagement feature 432. The first engagement feature 431 of the adapter 430 can be configured to releasably engage with the cap 412 of the container assembly 410. The second engagement feature 432 of the adapter 430 can be configured to releasably engage with the engagement feature 422 of the fluid access assembly 420.

The container size adapter 440 can include a first outer diameter portion 442A, a second outer diameter portion 442B, and a base 444. The first outer diameter portion 442A can have a smaller outermost diameter than the second outer diameter portion 442A. The first outer diameter portion 442A can be shaped and sized to be received in the first portion 421A of the interior 421. The second outer diameter portion 442B can be shaped and sized to be received in the second portion 421B of the interior 421. The first outer diameter portion 442A, the second outer diameter portion 442B, and the base 444 can collectively define a through-hole through which a container assembly can be translated into engagement with the fluid access component 424. The base 444 can extend radially relative to a central axis of the housing 428 beyond the outer diameter of the second outer diameter portion 442B. The base 444 can have a circular outer edge. The container size adapter 440 can be coupled to the fluid access assembly 420 as shown in FIG. 4A such that the through-hole of the container size adapter 440 can support a smaller size container assembly 410 having a cylindrical shape.

The cap 412 can include a resealable membrane such that a fluid access component, such as fluid access component 424, can pierce the resealable membrane to achieve fluidic communication with the reservoir 411. The resealable membrane of the cap 412 can be configured to reseal upon decoupling the fluid access component 424 from the cap 412 such that the reservoir 411 is fluidically isolated from an area external to the container assembly 410. Furthermore, the reservoir 411 can be an evacuated reservoir such that, when the reservoir 411 is in fluidic communication with a source of fluid (e.g., via piercing the resealable membrane of the cap 412 with a fluid access component fluidically coupled to a patient's vasculature), fluid can be drawn into the reservoir 411 due to a pressure differential between the reservoir 411 and the source of fluid. As shown in FIG. 4B, the cap 412 can include ridges 418 and an outwardly projecting annular flange 419 disposed on an external surface of the cap 412. The cap 412 can be formed of, for example, rubber. The tube 414 can be formed of, for example, plastic.

The fluid access assembly 420 can have a first end 423 and a second end 425. The fluid access component 424 can be disposed within the interior 421 of the housing 428 and can extend from the first end 423 of the housing 428 into the interior 421 of the housing 428. For example, in some embodiments, the fluid access component 424 can have a first end and a second end opposite the first end, the first end of the fluid access component 424 coupled to the first end of the housing 428 and the second end of the fluid access component 424 disposed in the interior 421 of the housing 428. The fluid access component 424 can include a needle defining a lumen.

The fluid connector component 426 can be coupled to the first end 423 of the housing 428. For example, the fluid connector component 426 can be coupled to the housing 428 and can define an outlet fluidically coupled to the lumen of the fluid access component 424. The fluid connector component 426 can include any suitable component configured to couple the housing 428 to patient access tubing such that the lumen of the fluid access component 424 is in fluidic communication with, for example, a patient vasculature system. In some embodiments, the fluid connector component 326 can be a Luer connector.

The engagement feature 422 of the fluid access assembly 420 can include a flange extending perpendicularly from a central axis of the housing 428. The engagement feature 422 can form a surface of the fluid access assembly 420 and can be disposed in a plane containing the second end of the housing 428. As shown in FIG. 4B, the engagement feature 422 can include portions (i.e., flange extensions 427A) that extend farther from the central axis of the housing 428 than other portions (i.e., flange portions 427B) of the engagement feature 422 extend from the central axis of the housing 428. The flange extensions 427A can be oppositely disposed and can each have a curved outer edge. The flange portions 427B can be oppositely disposed and can each have a straight outer edge. The flange extensions 427A can be separated from each other by the flange portions 427B. In some embodiments, the fluid access assembly 420 can be any suitable standard holder and the container size adapter 440 can be any suitable container size adapter, such as, for example, a JELCO^{®} Saf-T Holder^{®} device sold by Smiths Medical.

The first engagement feature 431 of the adapter 430 can be configured to releasably engage with the cap 412 of the container assembly 410. The first engagement feature 431 of the adapter 430 can include an inner surface of the adapter 430, the inner surface defining a through-hole. A central axis of the through-hole can be configured to be coaxial with a central axis of the housing 428 of the fluid access assembly 420. The inner surface of the adapter 430 can have a diameter sufficiently small relative to an outermost diameter of the outwardly projecting annular flange 419 of the cap 412 such that the inner surface and the cap 412 can be engaged via a friction fit. In some embodiments, the inner surface of the adapter 430 can have a diameter sufficiently small relative to an outermost diameter of the ridges 418 the cap 412 such that the inner surface and the ridges 418 of the cap 412 can be engaged via a friction fit.

The second engagement feature 432 of the adapter 430 can be configured to releasably engage with the engagement feature 422 of the fluid access assembly 420. For example, the second engagement feature 432 can include three arms or tabs 435 extending laterally away from a central axis of the housing 428. Each arm 435 has a first end coupled to a base 438 of the adapter 430. The second end of each curved arm 435 includes a latch 434, as can be seen in FIGS. 4A and 4B. The latches 434 can be configured to receive the outer edge of the base 444 of the container size adapter 440 and the flange extensions 427A and/or the flange portions 427B and can be separated from the flange extensions 427A and/or the flange portions 427B via pulling a tab 434A laterally outward relative to a central axis of the housing such that the latch 434 disengages the outer edge of the base 444 and the flange extensions 427A and/or the flange portions 427B. The adapter 430 can then be separated from the fluid access assembly 420 and the container assembly 410.

Although not shown, in some embodiments, rather than separating an adapter from a fluid access assembly via rotation, unlatching, or deforming (e.g., bending), the adapter can be broken such that the adapter and the container assembly can be removed. For example, the adapter can include perforations such that the adapter can be configured to break at certain locations for separation.

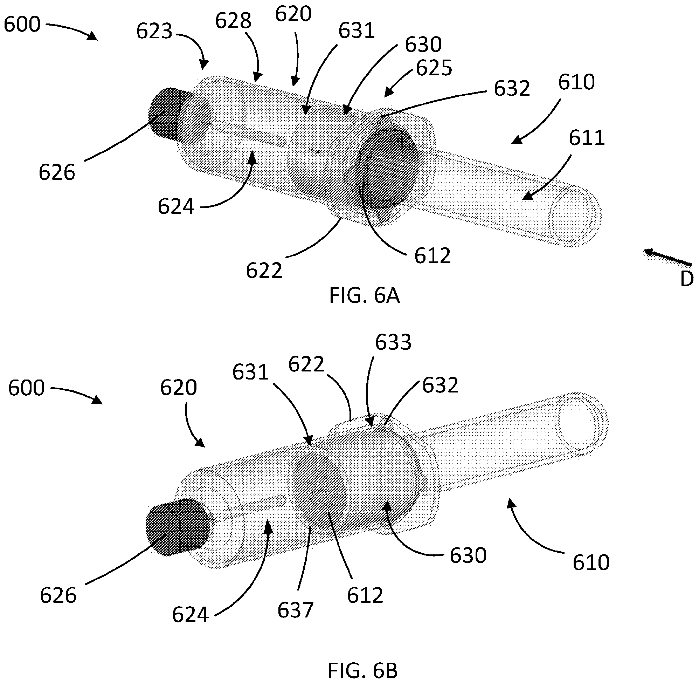

In some embodiments, an adapter may include a number of tabs that are configured to deform (e.g., bend) and/or break such that the adapter may be translated (e.g., with a container assembly) relative to a fluid access assembly. For example, FIGS. 6A and 6B are a proximal perspective view and a distal perspective view of a system 600, respectively. The system 600 may be similar in structure and/or function to any of the systems described herein, such as, for example, the system 100 and/or the system 200. For example, the system 600 includes a container assembly 610, a fluid access assembly 620, and an adapter 630. The container assembly 610 may be the same or similar in structure and/or function to the container assembly 110 and/or the container assembly 210. For example, the container assembly 610 may include a tube 614 and a cap 612. The tube 614 and the cap 612 may collectively define a reservoir 611. The fluid access assembly 620 can be the same or similar in structure and/or function to the fluid access assembly 110 and/or the fluid access assembly 210. For example, the fluid access assembly 620 may include a housing 628 defining an interior and having a first end 623 and a second end 625. The fluid access assembly 620 may also include a fluid access component 624, a fluid connector component 626, and an engagement feature 622.

The adapter 630 can be similar to any of the adapters described herein, such as the adapter 130 and/or the adapter 230 described above. For example, the adapter 630 can include a first engagement feature or portion including an inner surface of the adapter 630 and defining an opening configured to receive a cap 612 of the container assembly 610. The inner surface of the adapter 630 may have a diameter sufficiently small relative to the outermost diameter of the cap 612 (e.g., an annular flange of the cap 612 and/or ridges of the cap 612) such that the inner surface of the adapter 630 and the outer surface of the cap 612 can be engaged via a friction fit. The adapter 630 may have a first end 631 and a second end 633 opposite the first end 631. The adapter 630 may include an inwardly projecting flange 637 disposed near or adjacent to the first end 631 of the adapter 630. The inwardly projecting flange 637 may be configured to prevent the container assembly 610 from being translated in the direction of arrow D (e.g., toward the first end 623 of the housing 628) relative to the adapter 630 due to the engagement between the cap 612 and the inwardly projecting flange 637.

As shown in FIGS. 6A and 6B, the adapter 630 may include a second engagement feature including a number of tabs 632 configured to extend laterally from a central axis of the adapter 630 (e.g., outwardly) in a first configuration. Although four tabs 632 are shown, the adapter 630 may include any suitable number of tabs (e.g., one, two, three, five, or more). In some embodiments, when in the first configuration, the tabs 632 may be configured to engage with the engagement feature 622 (e.g., a proximal flange) of the fluid access assembly 620 and maintain the adapter 630 in a position relative to the fluid access component 624 such that the first end 631 of the adapter 630 is spaced from a fluid access component 624 of the fluid access assembly 620. In some embodiments, in the first configuration, the tabs 632 are configured to engage with an engagement feature 622 of the fluid access assembly 620 (e.g., a proximal flange) and maintain the adapter 630 such that the reservoir 611 of the container assembly 610 is fluidically isolated from the fluid access component 624. The tabs 632 may be configured to deform (e.g., bend or, flex) and/or break such that, when a force above a threshold force is applied to the adapter 630 in the direction of arrow D (e.g., via pushing the adapter 630 toward the first end 623 of the housing 628), the tabs 632 may bend or break such that the adapter 630 and the cap 612 may be translated toward the fluid access component 624. In some embodiments, the tabs 632 may include any suitable feature (e.g., a perforation or a pre-snap) such that the tabs 632 predictably bend or break.

The system 600 may have a first configuration (e.g., an initial configuration) in which the first engagement feature of the adapter 630 is engaged with the cap 612 and the tabs 632 are engaged with the engagement feature 622 of the fluid access assembly 620. When the first engagement feature of the adapter 630 is engaged with the cap 612 and the second engagement feature 632 is engaged with the engagement feature 622 of the fluid access assembly 620, the cap 612 may be spaced away from the fluid access component 624 such that the reservoir 611 is fluidically isolated from an external environment of the container assembly 610. In the first configuration, the cap 612 can be at least partially disposed within the adapter 630 and/or the housing 628.

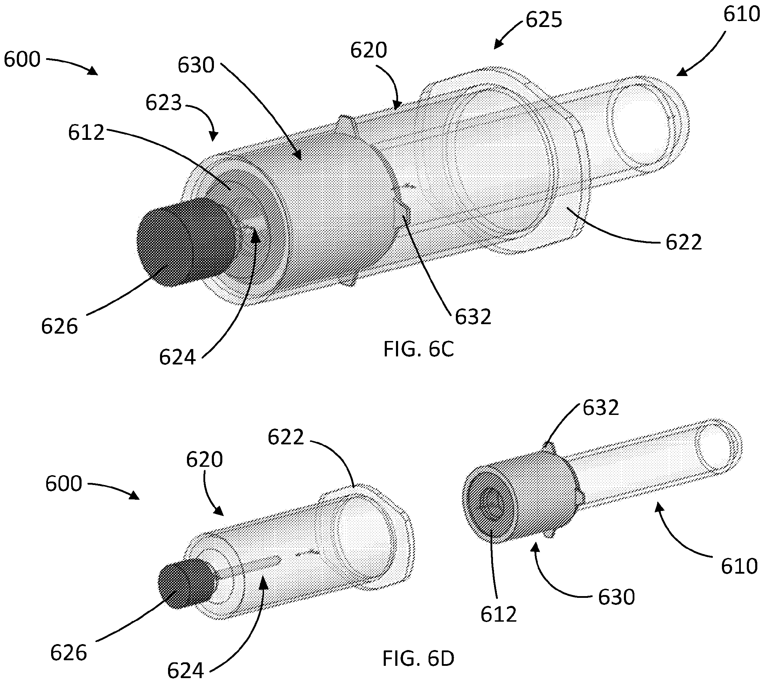

As shown in FIG. 6C, which is a perspective view of the system 600 in a second configuration, the system 600 may have a second configuration in which a lumen of the fluid access component 624 is in fluidic communication with the reservoir 611. To transition the system 600 from the first configuration to the second configuration, the container assembly 610 can be translated toward the first end 623 of the housing 628 of the fluid access assembly 620 such that the cap 612 applies a force to the adapter 630 such that the tabs 632 bend or break. The cap 612 may then be further translated into engagement with the fluid access component 624 (e.g., the fluid access component 624 pierces the cap 612 and a portion of the fluid access component 624 is disposed within the reservoir 611). Although FIG. 6C shows the tabs 632 as projecting outwardly from the adapter 630 in the second configuration, the tabs 632 may be bent proximally or detached from the remained of the adapter 630 in the second configuration. In the second configuration, when the fluid connector component 626 is fluidically coupled to a patient's vasculature system, fluid (e.g., blood) can be drawn through the fluid connector component 626, through the fluid access component 624, and into the reservoir 611 of the container assembly 610.

When sufficient blood has been drawn into the reservoir 611, the system 600 can be transitioned from the second configuration to a third configuration in which the container assembly 610 and the adapter 630 are separated from the fluid access assembly 620. For example, the container assembly 610 can be translated relative to the fluid access assembly 620 such that the cap 612 is disposed near the second end 625 of the housing 628. In response to the cap 612 being disengaged from the fluid access component 624, the reservoir 611 may be fluidically isolated from an environment external to the container assembly 610 (e.g., due to the cap 612 having a resealable membrane). The adapter 630 can then be decoupled from the fluid access assembly 620 via, further translating the container assembly 610 away from the first end 623 of the housing 628 as shown in FIG. 6D. The adapter 630 and the container assembly 610 may then be optionally discarded.

After removing the adapter 630 and the container assembly 610 from the fluid access assembly 620, a second container assembly can be inserted into the interior of the housing 628 and engaged with the fluid access component 624 such that the second container assembly can draw fluid (e.g., blood) into a reservoir of the second container assembly via the fluid connector component 626 and the fluid access component 624. In some embodiments, the second container assembly can include a medium (e.g., a soybean casein digest broth) in the reservoir of the second container configured to be used to perform a blood culture when combined with the patient's blood sample in the reservoir. Any suitable number of container assemblies can be engaged with the fluid access component 624 to draw fluid from a patient for various tests.

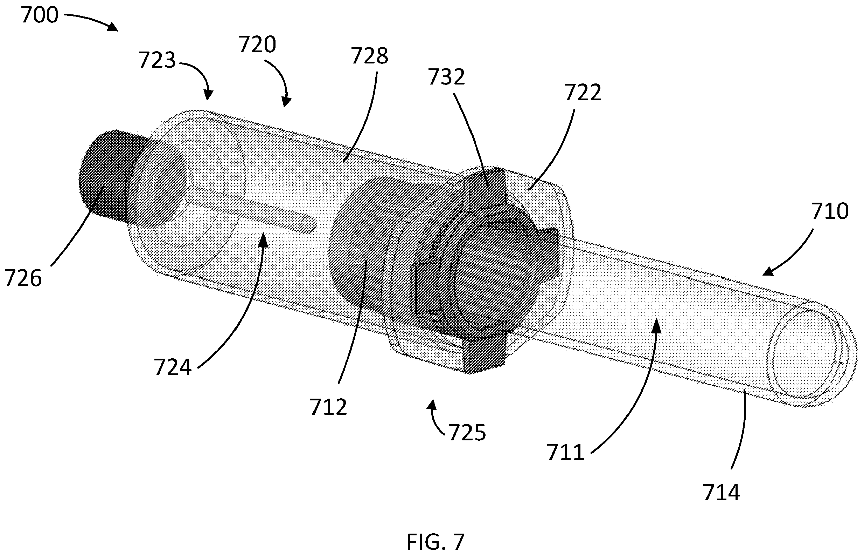

In some embodiments, a system does not include an adapter and instead the container assembly may include one or more engagement features configured to engage directly with an engagement feature of a fluid access assembly. For example, FIG. 7 is a perspective view of a system 700. The system 700 may be similar in structure and/or function to any of the systems described herein, such as, for example, the system 100 and/or the system 200. For example, the system 700 includes a container assembly 710 and a fluid access assembly 720. The container assembly 710 can be the same or similar in structure and/or function to the container assembly 710 and/or the container assembly 710. For example, the container assembly 710 may include a tube 714 and a cap 712. The tube 714 and the cap 712 may collectively define a reservoir 711. The fluid access assembly 720 can be the same or similar in structure and/or function to the fluid access assembly 110 and/or the fluid access assembly 210. For example, the fluid access assembly 720 may include a housing 728 defining an interior and having a first end 723 and a second end 725. The fluid access assembly 720 may also include a fluid access component 724, a fluid connector component 726, and an engagement feature 722 (e.g., a proximal flange).

The fluid access assembly 720 can include a number of tabs 732. The tabs 732 may be the same or similar in structure and/or function to the tabs 632 describe above with reference to the system 600. For example, the tabs 732 may extend laterally from a central axis of the cap 712 (e.g., outwardly) in a first configuration. Although four tabs 732 are shown, the cap 712 may include any suitable number of tabs (e.g., one, two, three, five, or more). In some embodiments, when in the first configuration, the tabs 732 may be configured to engage with the engagement feature 722 (e.g., a proximal flange) of the fluid access assembly 720 and maintain the cap 712 in a position relative to the fluid access component 724 such that the cap 712 is spaced from a fluid access component 724 of the fluid access assembly 720 (i.e., such that the fluid access component 724 does not contact the cap 712). In some embodiments, in the first configuration, the tabs 732 are configured to engage with an engagement feature 722 of the fluid access assembly 720 (e.g., a proximal flange) and maintain the cap 712 such that the reservoir 711 of the container assembly 710 is fluidically isolated from the fluid access component 724. The tabs 732 may be configured to deform (e.g., bend or flex) and/or break such that, when a force above a threshold force is applied to the cap 712 in the direction extending from the second end 725 to the first end 723 of the housing 728 (e.g., via pushing the cap 712 toward the first end 723 of the housing 728), the tabs 732 may bend or break due to the force on the container assembly 710 toward the first end 723 of the housing 728 being greater than the force of the engagement feature 722 on the tabs 732 (which may be a force holding the housing 728 stationary) such that the cap 712 may be translated toward the fluid access component 724. In some embodiments, the tabs 732 may include any suitable feature (e.g., a perforation or a pre-snap) such that the tabs 732 predictably bend or break.

The system 700 may have a first configuration (e.g., an initial configuration) in which the tabs 732 are engaged with the engagement feature 722 of the fluid access assembly 720. When the tabs 732 are engaged with the engagement feature 722 of the fluid access assembly 720, the cap 712 may be spaced away from the fluid access component 724 such that the cap 712 is not in contact with the fluid access component 724 and the reservoir 711 is fluidically isolated from an external environment of the container assembly 710. In the first configuration, the cap 712 can be at least partially disposed within the housing 728.

The system 700 may have a second configuration in which a lumen of the fluid access component 724 is in fluidic communication with the reservoir 711. To transition the system 700 from the first configuration to the second configuration, the container assembly 710 can be translated toward the first end 723 of the housing 728 of the fluid access assembly 720 such that the tabs 732 bend or break relative to the cap 712. For example, the tabs 732 may be flexed such that the tabs 732 extend away from the cap 712 a shorter lateral distance than the distance the tabs 732 extend when the system 700 is in the first configuration. The tabs 732 may be configured to translate with the cap 712 within the housing 728 when bent or flexed. The cap 712 may then be further translated into engagement with the fluid access component 724 (e.g., the fluid access component 724 pierces the cap 712 and a portion of the fluid access component 724 is disposed within the reservoir 711). In the second configuration, when the fluid connector component 726 is fluidically coupled to a patient's vasculature system, fluid (e.g., blood) can be drawn through the fluid connector component 726, through the fluid access component 724, and into the reservoir 711 of the container assembly 710.

When sufficient blood has been drawn into the reservoir 711, the system 700 can be transitioned from the second configuration to a third configuration in which the container assembly 710 and the adapter 730 are separated from the fluid access assembly 720. For example, the container assembly 710 can be translated relative to the fluid access assembly 720 such that the cap 712 is disposed near the second end 725 of the housing 728. In response to the cap 712 being disengaged from the fluid access component 724, the reservoir 711 may be fluidically isolated from an environment external to the container assembly 710 (e.g., due to the cap 712 having a resealable membrane). The cap 712 can then be decoupled from the fluid access assembly 720 via further translating the container assembly 710 away from the first end 723 of the housing 728. The adapter 730 and the container assembly 710 may then be optionally discarded.

After removing the container assembly 710 from the fluid access assembly 720, a second container assembly can be inserted into the interior of the housing 728 and engaged with the fluid access component 724 such that the second container assembly can draw fluid (e.g., blood) into a reservoir of the second container assembly via the fluid connector component 726 and the fluid access component 724. In some embodiments, the second container assembly can include a medium (e.g., a soybean casein digest broth) in the reservoir of the second container configured to be used to perform a blood culture when combined with the patient's blood sample in the reservoir. Any suitable number of container assemblies can be engaged with the fluid access component 724 to draw fluid from a patient for various tests.

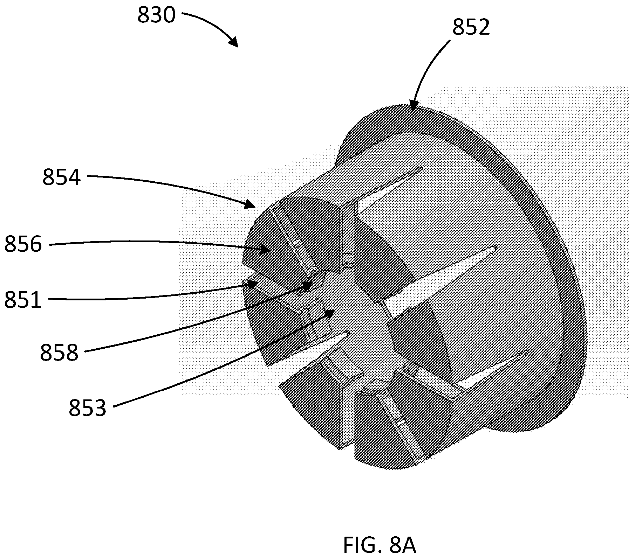

In some embodiments, a system may include an adapter including flexible wings configured to engage with a cap of a container assembly. For example, FIGS. 8A-8E are various views of components of a system 800. Specifically, FIG. 8A is a perspective view of an adapter 830 of the system 800. The adapter 830 may include a proximal flange 852 (i.e., a first engagement feature) and a number of flexible wings 854. Each flexible wing 854 may include a distal end wall 856 and an interior wall 858. The interior walls 858 of each flexible wing 854 may by arranged to form at least a portion of opening 853. Each flexible wing 854 may be separated from and adjacent flexible wing 854 by a slot 851. The adapter 830 may include any suitable number of flexible wings 854.

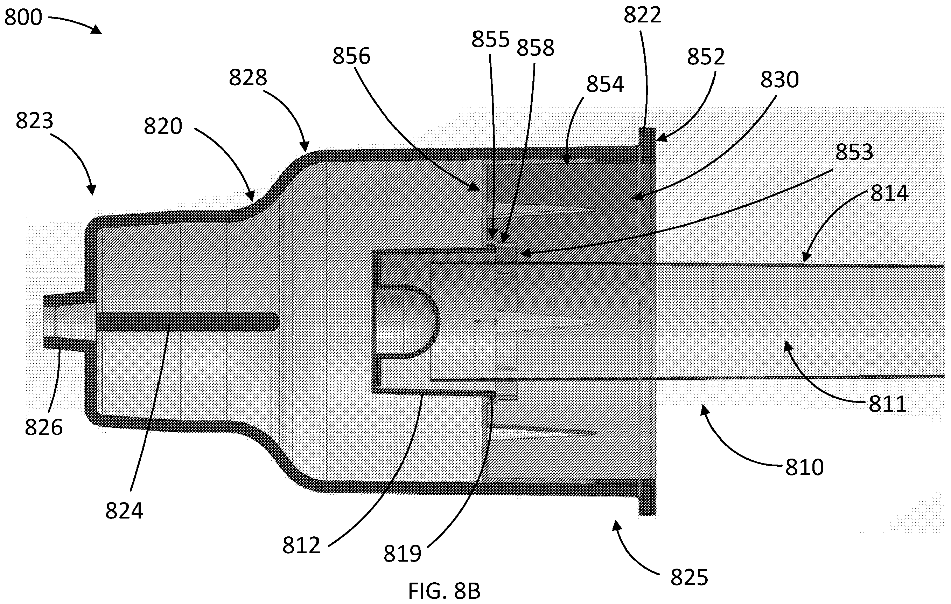

As shown in FIG. 8B, the system may also include a container assembly 810, a fluid access assembly 820, and an adapter 830. The system 800 may be similar in structure and/or function to any of the systems described herein, such as, for example, the system 100 and/or the system 200. For example, the container assembly 810 may be the same or similar in structure and/or function to the container assembly 110 and/or the container assembly 210. The container assembly 810 may include a tube 814 and a cap 812. The tube 814 and the cap 812 may collectively define a reservoir 811. The fluid access assembly 820 can be the same or similar in structure and/or function to the fluid access assembly 110 and/or the fluid access assembly 210. For example, the fluid access assembly 820 may include a housing 828 defining an interior and having a first end 823 and a second end 825. The fluid access assembly 820 may also include a fluid access component 824, a fluid connector component 826, and an engagement feature 822 (e.g., a proximal flange).

The adapter 830 can be similar to any of the adapters described herein, such as the adapter 130 and/or the adapter 230 described above. For example, the proximal flange 852 may engage with the engagement feature 822 of the fluid access assembly 820 such that the adapter 830 is prevented from translating toward the first end 823 of the fluid access assembly 820. The interior wall 858 of each flexible wing 854 of the adapter 830 is configured to engage and retain the cap 812 of the container assembly 810. For example, the interior wall 858 of each flexible wing 854 may define a groove 855 such that the grooves 855 of each flexible wing 854 substantially form a circular groove. A flange of the cap 812, such as an annular flange 819, may be configured to be received by the grooves 855 of the flexible wings 854. When the cap 812 is disposed within the opening 853 of the adapter 830, the flexible wings 854 may be pushed outwardly due to engagement with the cap 812 such that an outer surface of each flexible wing 854 is pushed into engagement with an inner surface of the housing 828. Thus, the container assembly 810 and the adapter 830 may be secured relative to the fluid access assembly 820. The engagement between the flange 830 and the first engagement portion 822 and between the flexible wings 854 and the cap 812 may prevent the container assembly 810 from being translated toward the first end 823 of the housing 828. Although the system 800 is shown an described as having a cap 812 including an annular flange 819 and flexible wings 854 having grooves 855, in some embodiments the cap 812 and the interior surfaces 858 of the flexible wings 854 may have corresponding tapers such that the cap 812 may be retained by the flexible wings 854.

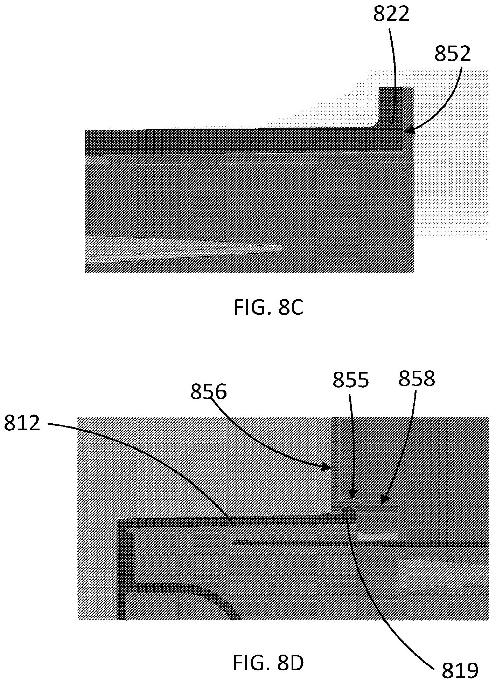

The system 800 may have a first configuration (e.g., an initial configuration) in which the proximal flange 852 of the adapter 830 is engaged with the first engagement portion 822 (as shown in FIG. 8C, which is a close up view of a first portion of FIG. 8B) and the cap 812 is engaged with the flexible wings 854 (as shown in FIG. 8D, which is a close up view of a second portion of FIG. 8B). In the first configuration, the cap 812 may be spaced away from the fluid access component 824 such that the reservoir 811 is fluidically isolated from an external environment of the container assembly 810. In the first configuration, the cap 812 can be at least partially disposed within the adapter 830 and/or the housing 828.

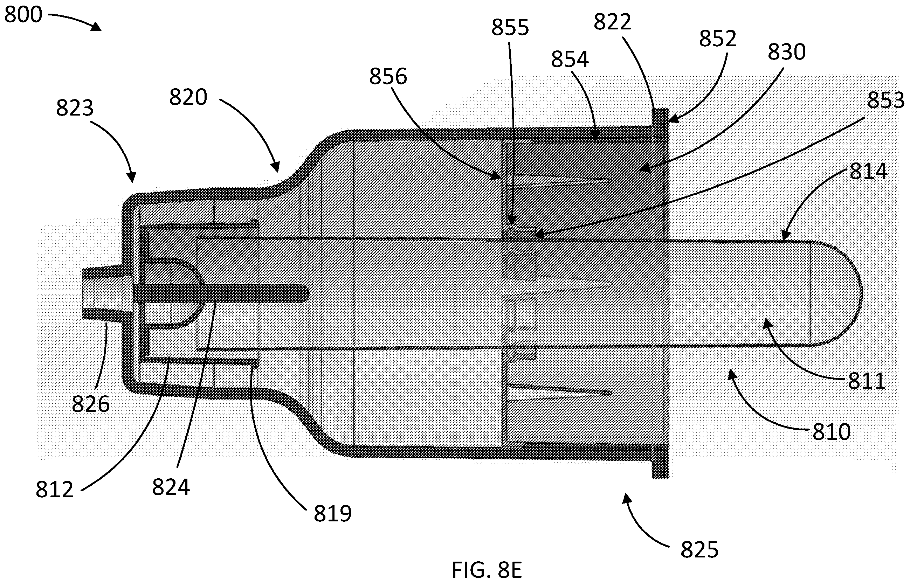

The system 800 may have a second configuration in which a lumen of the fluid access component 824 is in fluidic communication with the reservoir 811. To transition the system 800 from the first configuration to the second configuration, the container assembly 810 can be translated toward the first end 823 of the housing 828 of the fluid access assembly 820 such that the cap 812 applies a force to the adapter 830 such that the flexible tabs 832 bend and the cap 812 is translated beyond the adapter 830 toward the first end 823 of the housing 828. The cap 812 may then be further translated into engagement with the fluid access component 824 (e.g., the fluid access component 824 pierces the cap 812 and a portion of the fluid access component 824 is disposed within the reservoir 811). In the second configuration, when the fluid connector component 826 is fluidically coupled to a patient's vasculature system, fluid (e.g., blood) can be drawn through the fluid connector component 826, through the fluid access component 824, and into the reservoir 811 of the container assembly 810. As shown in FIG. 8E, when the cap 812 is engaged with the fluid access component 824, the tube 814 may be loosely situated in the opening 853 (e.g., due to the outer diameter of the tube 814 being smaller than the diameter of the opening 853 defined by the flexible wings 854).

When sufficient blood has been drawn into the reservoir 811, the system 800 can be transitioned from the second configuration to a third configuration in which the container assembly 810 and the adapter 830 are separated from the fluid access assembly 820. For example, the container assembly 810 can be translated relative to the fluid access assembly 820 such that the cap 812 is disposed near the second end 825 of the housing 828. In response to the cap 812 being disengaged from the fluid access component 824, the reservoir 811 may be fluidically isolated from an environment external to the container assembly 810 (e.g., due to the cap 812 having a resealable membrane). The adapter 830 can then be decoupled from the fluid access assembly 820 via, further translating the container assembly 810 away from the first end 823 of the housing 828 as shown in FIG. 8D. The adapter 830 and the container assembly 810 may then be optionally discarded.

After removing the adapter 830 and the container assembly 810 from the fluid access assembly 820, a second container assembly can be inserted into the interior of the housing 828 and engaged with the fluid access component 824 such that the second container assembly can draw fluid (e.g., blood) into a reservoir of the second container assembly via the fluid connector component 826 and the fluid access component 824. In some embodiments, the second container assembly can include a medium (e.g., a soybean casein digest broth) in the reservoir of the second container configured to be used to perform a blood culture when combined with the patient's blood sample in the reservoir. Any suitable number of container assemblies can be engaged with the fluid access component 824 to draw fluid from a patient for various tests.

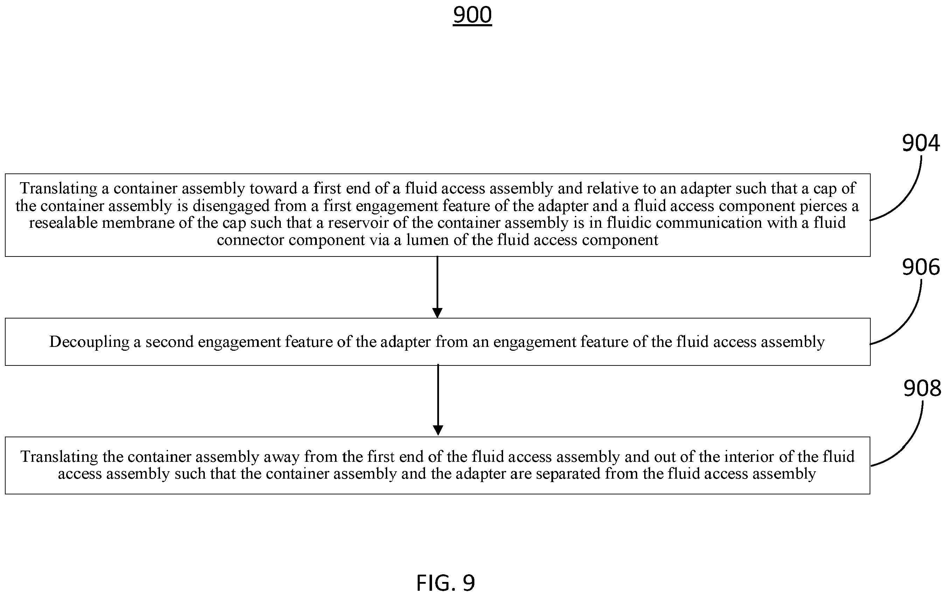

FIG. 9 is a flow chart illustrating a method 900. The method 900 can be performed using any suitable system or device, such as any of the systems of devices described herein. For example, the method 900 can be a method of using a system including, in an initial configuration, a container assembly having a cap and defining a reservoir, the container assembly having a first end and a second end, the cap disposed at the first end of the container assembly. The system can also include a fluid access assembly including a housing defining an interior, a fluid access component, a fluid connector component, and an engagement feature. The housing can have a first end and a second end and the fluid access component can extend from the first end of the housing into the interior of the housing. The fluid access component can define a lumen. The fluid connector component can be disposed on the first end of the housing and can be configured to be coupled to a patient access device such that the lumen of the fluid access component can be in fluidic communication with a patient's vasculature via the fluid connector component and the patient access device. The patient access device can include, for example, a needle and tubing. In some embodiments, the engagement feature of the fluid access assembly can be disposed on and/or coupled to the second end of the housing. The system can also include an adapter having a first engagement feature and a second engagement feature. The first engagement feature of the adapter can be releasably engaged with the cap of the container assembly and the second engagement feature of the adapter can be releasably engaged with the engagement feature of the fluid access assembly in the initial configuration such that the cap of the container assembly is at least partially disposed within the interior of the housing and spaced from the fluid access component.

The method 900 also includes translating the container assembly toward the first end of the fluid access assembly and relative to the adapter such that the cap is disengaged from the first engagement feature of the adapter and the fluid access component pierces a resealable membrane of the cap such that the reservoir of the container assembly is in fluidic communication with the fluid connector component via the lumen of the fluid access component, at 904. At 906, the second engagement feature of the adapter can be decoupled from the engagement feature of the fluid access assembly. In some embodiments, decoupling the second engagement feature of the adapter from the engagement feature of the fluid access assembly includes at least one of rotating, unlatching, and/or bending the adapter relative to the housing of the fluid access assembly. The container assembly can be translated away from the first end of the fluid access assembly and out of the interior of the fluid access assembly such that the container assembly and the adapter are separated from the fluid access assembly, at 906.

In some embodiments, prior to providing the system, the system can be sterilized. For example, in some embodiments, the system can be sterilized in the initial configuration. In some embodiments, the system can be sterilized prior to assembly into the initial configuration and the system can then be assembled in such a manner that sterility is maintained.

In some embodiments, the container assembly can be discarded after being separated from the fluid access assembly.

In some embodiments, the container assembly is a first container assembly and, after translating the first container assembly away from the first end of the fluid access assembly and out of the interior of the fluid access assembly, a second container assembly can be translated toward the first end of the fluid access assembly such that a resealable membrane of the second container assembly is pierced by the fluid access component and a reservoir of the second container assembly is in fluidic communication with the fluid connector component via the lumen of the fluid access component. In some embodiments, the reservoir of the second container assembly can include a medium configured to be combined with a blood sample to perform a blood culture.

In some embodiments, translating the container assembly toward the first end of the fluid access assembly and relative to the adapter such that the fluid access component pierces a resealable membrane of the cap causes the reservoir to draw fluid from a fluid source fluidically coupled to the fluid connector component, through the fluid access component, and into the reservoir of the container assembly due to the reservoir of the container assembly being evacuated. In some embodiments, the fluid source is a vasculature system of a patient.

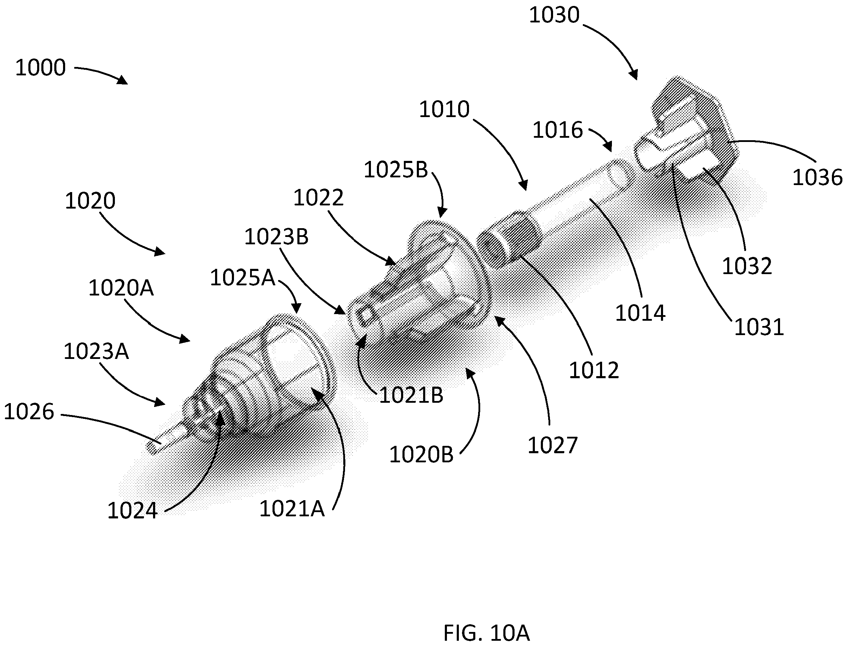

In some embodiments, a system may include an adapter configured to be rotationally and radially secured relative to a fluid access assembly. For example, FIGS. 10A-10I are various views of components of a system 1000. Specifically, FIG. 10A is an exploded perspective view of the system 1000. The system 1000 includes a container assembly 1010, a fluid access assembly 1020, and an adapter 1030. The system 1000 may be similar in structure and/or function to any of the systems described herein, such as, for example, the system 100 and/or the system 200. For example, the container assembly 1010 may be the same or similar in structure and/or function to the container assembly 110 and/or the container assembly 210. The container assembly 1010 can include a tube 1014 and a cap 1012. The tube 1014 and the cap 1012 can collectively define a reservoir.

The fluid access assembly 1020 can include a first housing portion 1020A and a second housing portion 1020B. The first housing portion 1020A can define an interior 1021A configured to receive the second housing portion 1020B. The second housing portion 1020B can define an interior 1021B. The fluid access assembly 1020 includes a fluid access component 1024 and a fluid connector component 1026. The second housing portion 1020B includes a plurality of engagement features 1022 protruding from a tubular portion of the second housing portion 1020B. Each engagement feature 1022 defines a recess extending away from a central axis of the second housing portion 1020B. As shown in FIG. 10A, the engagement features 1022 can each have a stepped outer profile corresponding to a stepped outer profile of an interior surface of the first housing portion 1020A such that the second housing portion 1020B can be received within the interior 1021A of the first housing portion 1020A with a flange 1027 disposed on a proximal end 1025B of the second housing portion 1020B disposed adjacent to a proximal end 1025A of the first housing portion 1020A (e.g., the end opposite the distal end 1023B of the first housing portion 1020A including the fluid connector component 1026).

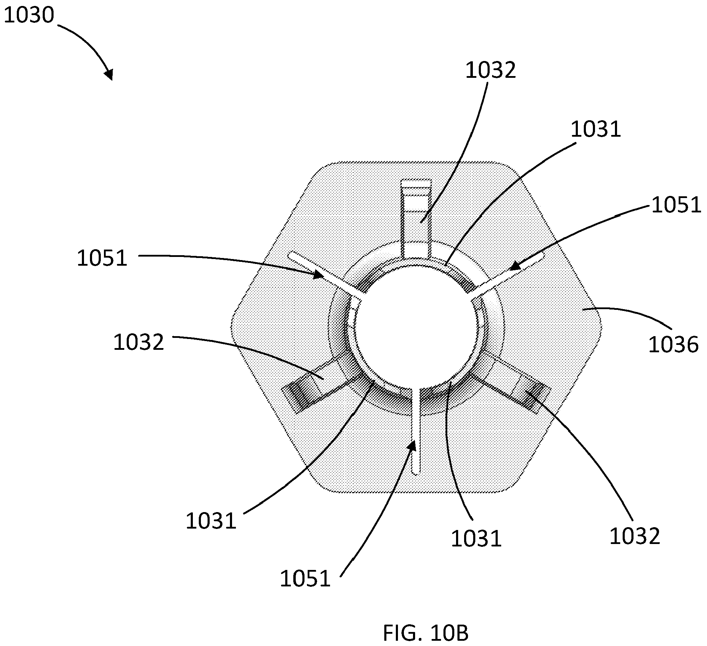

The adapter 1030 includes a proximal flange 1036 and a plurality of flexible tab portions 1031 (i.e., collectively forming a first engagement feature). The adapter 1030 also includes a plurality of projecting portions 1032 (i.e., second engagement features). Each projecting portion 1032 is coupled to and projects away from (e.g., radially extends relative to) a tab portion 1031 relative to a central axis of the adapter 1030. The flexible tab portions 1031 collectively form an opening configured to receive a portion of the container assembly 1010 (e.g., the cap 1012 of the container assembly 1010). The flexible tab portions 1031 can each include a curved inner surface configured to engage an outer surface of the cap 1012 of the container assembly 1010. As shown in FIG. 10B, which is a distal end view of the adapter 1030, each tab portion 1031 can be separated from an adjacent flexible tab portion 1031 by a slot 1051. Each slot 1051 can be any suitable shape (e.g., rectangular or triangular), and can extend from a free end of each tab portion 1031 to the interface (e.g., a hinge portion) between the tab portion 1031 and the flange 1036 and/or through a portion of the flange 1036 to increase the flexibility of the tab portion 1031 relative to the other tab portions 1031. When the adapter 1030 is in a neutral configuration (e.g., when the container assembly 1020 is not disposed within the opening through the adapter 1030 defined by the tab portions 1031), the tab portions 1031 can define a smaller diameter opening than when the adapter is engaged with the cap 1012 of the container assembly 1020. Although the second housing portion 1020B is shown as having three engagement features 1022 and the adapter 1030 is shown as having three projecting portions 1032, in some embodiments the second housing portion 1020B can have any suitable number of engagement features 1022 and the adapter 1030 can have any suitable number of projecting portions 1032. The adapter 1030 can be formed of any suitable material, such as, for example, polypropylene.

Each projecting portion 1032 can be configured to be received by an engagement feature 1022 of the second housing portion 1020B (e.g., when the adapter 1030 is inserted into the interior 1021B of the second housing portion 1020B such that the flange 1036 is adjacent to the flange 1027 of the second housing portion 1020B). In some embodiments, when the projecting portions 1032 are received within the recesses of the engagement features 1022 and the adapter 1030 is in the neutral configuration, a gap can be defined between an outer edge of the projecting portions 1032 and an inner surface of the engagement features 1022 such that the adapter 1030 is slidable relative to the second housing portion 1020B. When the cap 1012 is received within the opening defined by the tab portions 1031, the projecting portions 1032 can be disposed in contact with the inner surface of the engagement features 1022 such that the adapter 1030 is maintained in place relative to the second housing portion 1020B. In some embodiments, when the adapter 1030 is disposed in the interior 1021B of the second housing portion 1020B, the projecting portions 1032 can contact the inner surface of the engagement features 1022 in both the neutral configuration in which the cap 1012 is not disposed within the opening in the adapter 1030 and the configuration in which the cap 1012 is received within the opening in the adapter 1030, but the projecting portions 1032 apply a greater force on the inner surface of the engagement features 1022 when the cap 1012 is received within the opening in the adapter 1030 such that the adapter 1030 is maintained in place relative to the second housing portion 1020B.

In some embodiments, adapter 1030 can be in the neutral configuration both when the cap 212 is not disposed within the opening defined by the tab portions 1032 and when the cap 1012 is disposed within the opening defined by the tab portions 1032 such that the inner diameter of the opening is the same in both configurations of the container assembly 1010. The engagement features 1022 can define recesses that are sufficiently shallow relative to the central axis of the second housing portion 1020B (e.g., the distance between the inner surface of each engagement feature 1022 facing the central axis and the central axis is sufficiently small) such that, when the adapter 1030 is engaged with the second housing portion 1020B such that the projecting tabs 1030 are disposed within the recesses of the engagement features 1022, the inner surface of each engagement feature 1022 applies force to a projecting portion 1032 in the direction of the central axis such that the tab portions 1031 are pushed into tighter engagement with the cap 1012 such that the cap 1012 is immobilized relative to the adapter 1030 and the fluid access assembly 1020.

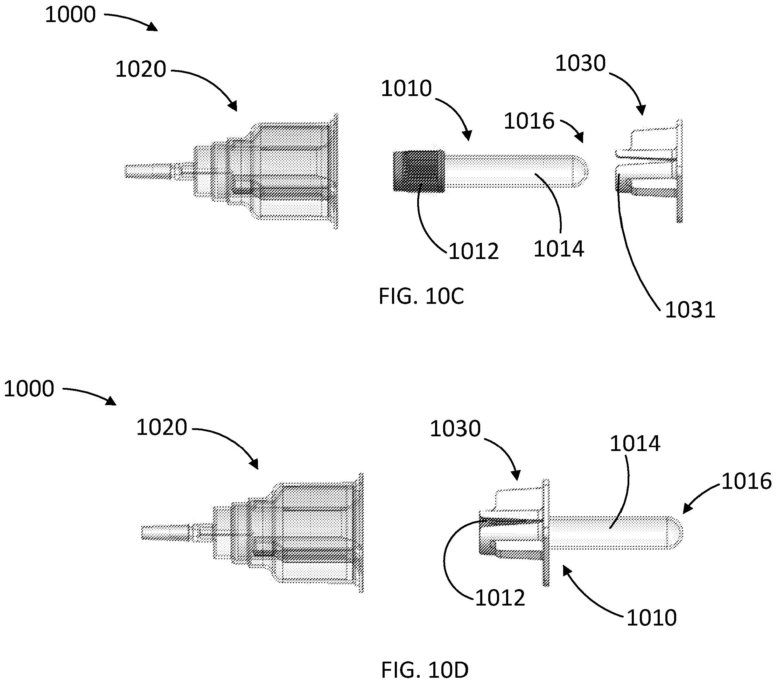

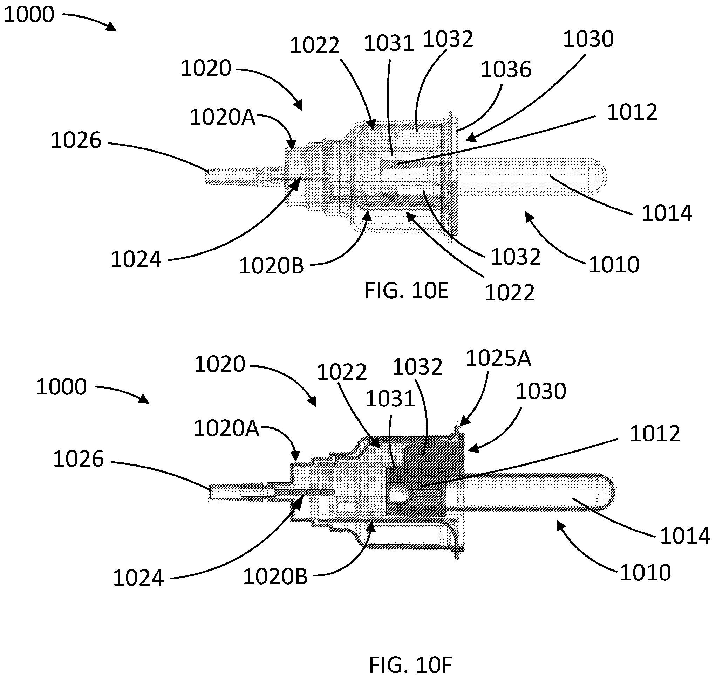

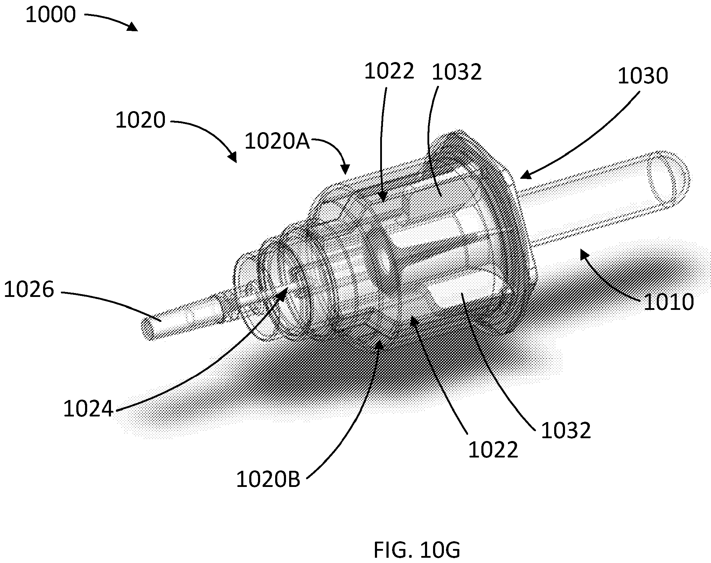

FIGS. 10C-10E are side views of the system 1000 in various stages of assembly, to assemble the system 1000 into a first configuration (e.g., an initial configuration shown in FIG. 10G, which is a perspective view). The second housing portion 1020B can be inserted into the interior 1021A of the first housing portion 1020A (as show in FIG. 10C). As shown in FIG. 10D, the adapter 1030 can then be coupled to the container assembly 1010 such that the cap 1012 is disposed within the opening of the adapter 1030 defined by the tab portions 1031. As shown in FIGS. 10E and 10F, which are a side view and cross-sectional side view of the system 1000, respectively, the adapter 1030 and the container assembly 1010 can then be inserted into the interior 1021B of the second housing portion 1020C such that the flange 1036 of the adapter 1030 is adjacent to the flange 1027 of the second housing portion 1020B and the projecting portions 1032 are disposed within the recesses defined by the engagement features 1022. As shown in FIG. 10F, in such a configuration (e.g., the first or initial configuration), the projecting portions 1032 are in contact with the inner surface of the engagement features 1022 such that the tab portions 1031 apply a retaining force to the cap 1012 due to the engagement between the inner surface of the engagement features 1022 and the projecting portions 1032. As shown in FIGS. 10E and 10F, in the initial configuration, the cap 1012 is spaced from the fluid access component 1024 (e.g., a shaft defining a lumen in fluid communication with the fluid connector component 1026) such that the reservoir of the container assembly 1010 is fluidically isolated from an external environment of the container assembly 1010. As shown in FIGS. 10E and 10F, the cap 1012 can be at least partially (e.g., fully) disposed within the adapter 1030 and/or the fluid access assembly 1020.

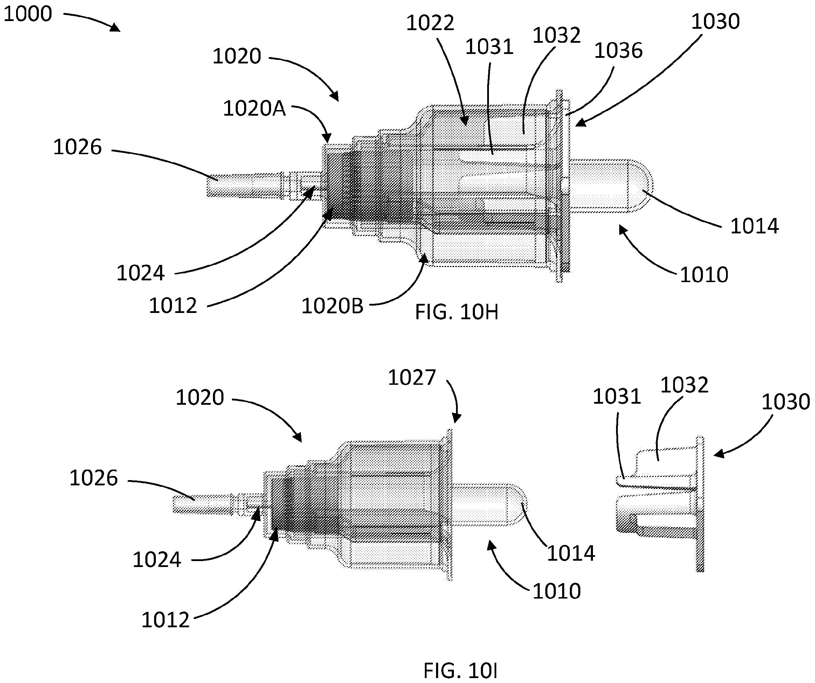

As shown in FIG. 10H, the system 1000 can have a second configuration in which the lumen of the fluid access component 1024 is in fluidic communication with the reservoir of the container assembly 1010. To transition the system 1000 from the first configuration to the second configuration, the container assembly 1010 can be translated toward the distal end 1023A of the first housing portion 1020A of the fluid access assembly 1020 by applying a translating force to the container assembly 1010 (e.g., to the closed end 1014 of the container assembly) that overcomes the retaining force applied by the tab portions 1031 on the cap 1012 such that the cap 1012 is translated beyond the adapter 1030 toward the first end 1023A of the first housing portion 1020A. The cap 1012 may then be further translated into engagement with the fluid access component 1024 (e.g., the fluid access component 1024 pierces the cap 1012 and a portion of the fluid access component 1024 is disposed within the reservoir of the container assembly 1010). In the second configuration, when the fluid connector component 1026 is fluidically coupled to a patient's vasculature system (e.g., after the cap shown in FIG. 10H is removed), fluid (e.g., blood) can be drawn through the fluid connector component 1026, through the fluid access component 1024, and into the reservoir of the container assembly 1010.

When sufficient blood has been drawn into the reservoir of the container assembly 1010, the system 1000 can be transitioned from the second configuration to a third configuration in which the container assembly 1010 and the adapter 1030 are separated from the fluid access assembly 1020. For example, as shown in FIG. 10I, when the cap 1012 is engaged with the fluid access component 1024, the adapter 1030 can be removed from the interior 1021B of the second housing portion 1020B (e.g., due to the cap 1012 no longer being disposed in the opening defined by the tab portions 1031 and applying a retaining force against the tab portions 1031 and the projecting portions 1032 against the inner surface of the engagement features 1022). The container assembly 1010 can be translated relative to the fluid access assembly 1020 such that the cap 1012 is disposed near the flange 1027 of the second housing portion 1020B. In response to the cap 1012 being disengaged from the fluid access component 1024, the reservoir of the container assembly 1010 may be fluidically isolated from an environment external to the container assembly 1010 (e.g., due to the cap 1012 having a resealable membrane). The container assembly 1010 can then be further translated away from the first end 1023A of the first housing portion 1020A and away from the first end 1023B of the second housing portion 1020B. The adapter 1030 and the container assembly 1010 may then be optionally discarded.

After removing the adapter 1030 and the container assembly 1010 from the fluid access assembly 1020, a second container assembly can be inserted into the interior 1021A of the first housing portion 1020A and engaged with the fluid access component 1024 such that the second container assembly can draw fluid (e.g., blood) into a reservoir of the second container assembly via the fluid connector component 1026 and the fluid access component 1024. In some embodiments, the second container assembly can be or include a blood culture bottle. In some embodiments, the second container assembly can include a medium (e.g., a soybean casein digest broth) in the reservoir of the second container assembly configured to be used to perform a blood culture when combined with the patient's blood sample in the reservoir. Any suitable number of container assemblies can be engaged with the fluid access component 1024 to draw fluid from a patient for various tests. Due to the arrangement of the first container assembly 1020 and the adapter 1030 relative to the fluid access assembly 1020 prior to engagement of the second container assembly with the fluid access component 1024 (e.g., in both the initial configuration and the second configuration), the fluid access assembly 1020, including the fluid access component 1024, can be accessed in a sterile manner by the second container assembly due to the interior of the fluid access assembly 1020, including the fluid access component 1024, being kept free from contamination prior to the second container assembly being engaged with the fluid access component 1024.

While various embodiments have been described above, it should be understood that they have been presented by way of example only, and not limitation. Where methods described above indicate certain events occurring in certain order, the ordering of certain events may be modified. Additionally, certain of the events may be performed concurrently in a parallel process when possible, as well as performed sequentially as described above.

Where schematics and/or embodiments described above indicate certain components arranged in certain orientations or positions, the arrangement of components may be modified. While the embodiments have been particularly shown and described, it will be understood that various changes in form and details may be made. Any portion of the apparatus and/or methods described herein may be combined in any combination, except mutually exclusive combinations. The embodiments described herein can include various combinations and/or sub-combinations of the functions, components, and/or features of the different embodiments described.

## Claims

1. A system (700), comprising:
a fluid access assembly (720) including a housing (728) defining an interior, a fluid access component (724), a fluid connector component (726), and an engagement feature (722), the housing (728) having a first end and a second end, the fluid access component (724) extending from the first end of the housing (728) into the interior of the housing (728), the fluid access component (724) defining a lumen, the fluid connector component (726) disposed on the first end of the housing (728) and configured to be coupled to a patient access device such that the lumen of the fluid access component (724) can be in fluidic communication with a patient's vasculature via the fluid connector component (726) and the patient access device, the engagement feature (722) of the fluid access assembly (720) disposed on the second end of the housing (728); and **characterized in that** the system further comprises:
a container assembly (710) including a cap (712) and an engagement feature (732), the container assembly defining a reservoir (711), the container assembly (710) having a first end and a second end, the cap (712) disposed at the first end of the container assembly (710), the engagement feature (732) of the container assembly (710) configured to releasably engage with engagement feature (722) of the fluid access assembly (720) such that, in a first configuration in which the engagement feature (732) of the container assembly (710) is engaged with the engagement feature (722) of the fluid access assembly (720), the cap (712) is at least partially disposed within the interior of the housing (728) and spaced from the fluid access component (724).

2. The system of claim 1, wherein the container assembly (710) is configured to be transitioned from the first configuration to a second configuration via translating the container assembly (710) toward the first end of the fluid access assembly (720) such that the engagement feature (732) of the container assembly (710) is disengaged from the engagement feature (722) of the fluid access assembly (720) and the fluid access component (724) pierces a resealable membrane of the cap (712) such that the reservoir (711) of the container assembly (710) is in fluidic communication with the fluid connector component (726) via the lumen of the fluid access component (724).

3. The system of claim 2, wherein the engagement feature (732) of the container assembly (710) is configured to separate from the cap (712) when the container assembly (710) is transitioned from the first configuration to the second configuration.

4. The system of claim 2, wherein the engagement feature (732) of the container assembly (710) is configured to deform relative to the cap when the container assembly (710) is transitioned from the first configuration to the second configuration.

5. The system of claim 1, wherein the engagement feature (732) of the container assembly (710) includes a plurality of tabs extending laterally away from a central axis of the container assembly (710).

6. The system of claim 1, wherein the engagement feature (732) of the container assembly (710) includes a plurality of tabs extending laterally away from a central axis of the container assembly (710).

7. The system of claim 1, wherein the engagement feature (732) of the container assembly (710) extends laterally from the cap in the first configuration.

8. The system of claim 1, wherein the engagement feature (722) of the fluid access assembly (720) includes a flange extending laterally away from a central axis of the housing (728).

9. A method of using a system, the system in an initial configuration including:
a fluid access assembly (720) including a housing (728) defining an interior, a fluid access component (724), a fluid connector component (726), and an engagement feature (722), the housing (728) having a first end and a second end, the fluid access component (724) extending from the first end of the housing (728) into the interior of the housing (728), the fluid access component (724) defining a lumen, the fluid connector component (726) disposed on the first end of the housing (728) and configured to be coupled to a patient access device such that the lumen of the fluid access component (724) can be in fluidic communication with a patient's vasculature via the fluid connector component (726) and the patient access device, the engagement feature (722) of the fluid access assembly (720) disposed on the second end of the housing (728); and
a container assembly (710) including a cap (712) and an engagement feature (732), the container assembly defining a reservoir (711), the container assembly (710) having a first end and a second end, the cap (712) disposed at the first end of the container assembly (710), the engagement feature (732) of the container assembly (710) configured to releasably engage with engagement feature (722) of the fluid access assembly (720) such that, in a first configuration in which the engagement feature (732) of the container assembly (710) is engaged with the engagement feature (722) of the fluid access assembly (720), the cap (712) is at least partially disposed within the interior of the housing (728) and spaced from the fluid access component (724);
wherein the method comprises:
translating the container assembly (710) toward the first end of the fluid access assembly (720) such that the engagement feature (732) of the container assembly (710) is disengaged from the engagement feature (722) of the fluid access assembly and the fluid access component (724) pierces a resealable membrane of the cap (712) such that the reservoir (711) of the container assembly (710) is in fluidic communication with the fluid connector component (726) via the lumen of the fluid access component (724); and
translating the container assembly (710) away from the first end of the fluid access assembly (720) and out of the interior of the fluid access assembly (720) such that the container assembly (710) is separated from the fluid access assembly (720).

10. A system (400), comprising:
a container assembly (410) including a cap (412) and a tube (414) defining a reservoir (411), the container assembly (410) having a first end and a second end, the cap (412) disposed at the first end of the container assembly (410);
a fluid access assembly (420) including a housing (428) defining an interior (421), a fluid access component (424), a fluid connector component (426), and an engagement feature (422), the housing (428) having a stepped outer profile such that the housing (428) includes a first inner diameter portion (428A) at a first end thereof and a second inner diameter portion (428B) at a second end thereof, the fluid access component (424) extending from the first end of the housing (428) into the interior of the housing (428), the fluid access component (424) defining a lumen, the fluid connector component (426) disposed on the first end of the housing (428) and configured to be coupled to a patient access device such that the lumen of the fluid access component (424) can be in fluidic communication with a patient's vasculature via the fluid connector component (426) and the patient access device; and
**characterized in that** the system further comprises:
an adapter (430) including a first engagement feature (431) and a second engagement feature (432), the first engagement feature (431) of the adapter (430) configured to releasably engage with the cap (412) of the container assembly (410) and the second engagement feature (432) of the adapter (430) configured to releasably engage with the engagement feature (422) of the fluid access assembly (420); and
a container size adapter (440) including a first outer diameter portion (442A), a second outer diameter portion (442B), and a base (444), the first outer diameter portion (442A) shaped and sized to be received in the first inner diameter portion (428A) and the second outer diameter portion (442B) shaped and sized to be received in the second inner diameter portion (428B), where the first outer diameter portion (442A), the second outer diameter portion (442B), and the base (444) collectively define a through-hole through which the container assembly (410) can be translated into engagement with the fluid access component (424), and where the second engagement feature (432) of the adapter (430) is configured to releasably engage with the base (444).

11. The system of claim 10, wherein the first inner diameter portion (428A) of the housing (428) has a smaller inner diameter than the second inner diameter portion of the housing (428), and wherein the first outer diameter portion (442A) of the container size adapter (440) has a smaller outermost diameter than the second outer diameter portion (442A) of the container size adapter (440).

12. The system of claim 10, wherein the base (444) extends radially relative to a central axis of the housing (428) and beyond an outer diameter of the second outer diameter portion (442B).

13. The system of claim 10, wherein the first engagement feature (431) of the adapter (430) includes an inner surface defining a through-hole coaxially aligned with a central axis of the housing (428), with the through-hole sized to engage the cap (412) via a friction fit.

14. The system of claim 10, wherein the second engagement feature (432) of the adapter (430) comprises a plurality of arms (435) extending laterally away from a central axis of the housing (428), with each arm (435) having a first end coupled to a base (438) of the adapter (430) and a second end including a latch (434) configured to receive the base (444) of the container size adapter (440) and the engagement feature (422) of the fluid access assembly (420).

15. The system of claim 10, wherein the adapter (430) is separable from the fluid access assembly (420) and the container assembly (410).
